(19) Europäisches Patentamt
European Patent Office
Office européen des brevets

(11) Veröffentlichungsnummer: **0 096 006**
**A2**

# (12) EUROPÄISCHE PATENTANMELDUNG

(21) Anmeldenummer: **83810218.4**

(22) Anmeldetag: **24.05.83**

(51) Int. Cl.³: **C 07 D 233/36**, C 07 D 403/12, C 07 D 417/12, C 07 C 127/15, A 61 K 31/415, A 61 K 31/535, A 61 K 31/425, A 61 K 31/17

(30) Priorität: **28.05.82 US 383049**

(43) Veröffentlichungstag der Anmeldung: **07.12.83 Patentblatt 83/49**

(84) Benannte Vertragsstaaten: **AT BE CH DE FR GB IT LI LU NL SE**

(71) Anmelder: **CIBA-GEIGY AG, Patentabteilung Postfach, CH-4002 Basel (CH)**

(72) Erfinder: **Werner, Lincoln Harvey, Dr., 94 Larned Road, Summit N.J. 07901 (US)**
Erfinder: **Ford, Neville, Dr., 7146A Dartmouth Avenue, University City Missouri 63130 (US)**

## (54) 3-(Ureidocyclohexylamino)-propan-1,2-diolderivate, Verfahren zu ihrer Herstellung, pharmazeutische Präparate enthaltend diese Verbindungen, sowie ihre therapeutische Verwendung.

(57) Die Erfindung betrifft kardiocaskulär wirkende Verbindungen der Formel I

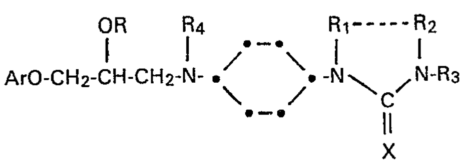

(I),

worin Ar einen (monocyclischen oder gegebenenfalls partiell hydrierten bicyclischen) carbocyclischen oder heterocyclischen, gegebenenfalls substituierten aromatischen Rest bedeutet, R für Wasserstoff, Alkanoyl oder Aroyl steht, $R_1$ und $R_2$ unabhängig voneinander Wasserstoff oder Niederalkyl oder $R_1$ und $R_2$ gemeinsam Alkylen mit 2 bis 7 Kohlenstoffatomen, welche die zwei Stickstoffatome durch 2 bis 4 Kohlenstoffatome trennen, bedeuten, $R_3$ und $R_4$ unabhängig voneinander für Wasserstoff oder Niederalkyl stehen, und X Oxo (O) oder Thio (S) bedeutet, ihre Salze, insbesondere therapeutisch verwendbaren Salze, sowie Verfahren zu ihrer Herstellung, pharmazeutische Präparate enthaltend diese Verbindungen, sowie ihre therapeutische Verwendung. Sie können z.B. durch Reduktion einer der Formel I entsprechenden Verbindung, worin anstelle des 1,4-Cyclohexylenrestes ein 1,4-Phenylenrest steht, hergestellt werden.

EP 0 096 006 A2

CIBA-GEIGY AG
Basel (Schweiz)

4-13936/+

# 3-(Ureidocyclohexylamino)-propan-1,2-diolderivate, Verfahren zu ihrer Herstellung, pharmazeutische Präparate enthaltend diese Verbindungen, sowie ihre therapeutische Verwendung

Die Erfindung betrifft neue Verbindungen der Formel I

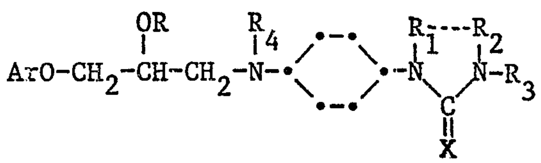

(I) ,

worin Ar einen (monocyclischen oder gegebenenfalls partiell hydrierten bicyclischen) carbocyclischen oder heterocyclischen, gegebenenfalls substituierten aromatischen Rest bedeutet, R für Wasserstoff, Alkanoyl oder Aroyl steht, $R_1$ und $R_2$ unabhängig voneinander Wasserstoff oder Niederalkyl oder $R_1$ und $R_2$ gemeinsam Alkylen mit 2 bis 7 Kohlenstoffatomen, welche die zwei Stickstoffatome durch 2 bis 4 Kohlenstoffatome trennen, bedeuten, $R_3$ und $R_4$ unabhängig voneinander für Wasserstoff oder Niederalkyl stehen, und X Oxo (O) oder Thio (S) bedeutet, ihre Salze, insbesondere therapeutisch verwendbaren Salze, sowie Verfahren zu ihrer Herstellung, pharmazeutische Präparate enthaltend diese Verbindungen, sowie ihre therapeutische Verwendung.

Bevorzugt sind die obigen Verbindungen der Formel I, worin Ar gegebenenfalls substituiertes [Phenyl, Pyridyl, Naphthyl, Tetrahydronaphthyl, 3,4-Dihydro-1(2H)-naphthalenon, 3,4-Dihydro-2-(1H)chinolon, 3,4-Dihydro-1(2H)-isochinolon, Indolyl oder 1,2,5-Thiadiazolyl] bedeutet,und ihre Salze, insbesondere ihre therapeutisch verwendbaren Salze.

Sehr bevorzugt sind Verbindungen der Formel I, worin Ar 1-Naphthyl, 1(2H)-Oxo-3,4-dihydronaphth-5-yl, 5,6,7,8-Tetrahydro-cis-6,7-dihydroxy-

BAD ORIGINAL

naphth-1-yl, 4-Indolyl, 3,4-Dihydro-2(1H)-chinolon-5-yl, 3-Cyano-2-pyridyl oder 4-(4-Morpholinyl)-1,2,5-thiadiazol-3-yl bedeutet, und ihre Salze, insbesondere ihre therapeutisch verwendbaren Salze.

Besonders bevorzugt sind Verbindungen der Formel I, worin Ar gegebenenfalls durch einen bis drei Substituenten der Gruppe niederes (Alkyl, Alkenyl, Alkinyl, Alkanoyl, Mono- oder Dialkylamino, Alkanoylamino, Alkylsulfonylamino, Alkoxycarbonyl, Alkylcarbamoyl, Alkylsulfamoyl, Alkoxy, Alkylthio, Alkylsulfinyl, Alkylsulfonyl, Alkenyloxy oder Alkinyloxy), Hydroxy, Cyano, Halogen, Pyrrolyl, Amino, 5-, 6- oder 7-gliedriges (Alkylen-, Oxaalkylen-, Thiaalkylen)imino, Benzyloxy, Phenyl, 5-, 6- oder 7-gliedriges Cyclalkyl, Carbamoyl, Sulfamoyl oder durch 3- bis 7-gliedriges Cycloalkyl-, Phenyl-, Hydroxy-, Niederalkoxy-, Niederalkoxycarbonylamino-, Niederalkylthio-, Niederalkylsulfinyl-, Niederalkylsulfonyl- und durch Carbamoyl-substituiertes (Niederalkyl oder Niederalkoxy) substituiertes Phenyl bedeutet, R für Wasserstoff, Niederalkanoyl oder Aroyl steht; $R_1$ und $R_2$ Wasserstoff oder Niederalkyl, oder $R_1$ und $R_2$ gemeinsam Alkylen mit 2 bis 5 Kohlenstoffatomen bedeuten, $R_3$ und $R_4$ für Wasserstoff oder Niederalkyl stehen und X O oder S ist und ihre Salze, insbesondere ihre therapeutisch verwendbaren Salze.

Ganz besonders bevorzugt sind die oben erwähnten Verbindungen der Formel I, worin R Wasserstoff bedeutet; $R_1$ und $R_2$ gemeinsam für Alkylen mit 2 bis 4 Kohlenstoffatomen stehen, wodurch sich 5-, 6- oder 7-gliedrige Ringe bilden; $R_3$ und $R_4$ Wasserstoff oder Niederalkyl bedeuten, und X O oder S ist, und ihre Salze, insbesondere ihre therapeutisch verwendbaren Salze.

In erster Linie bevorzugt sind Verbindungen der Formel II

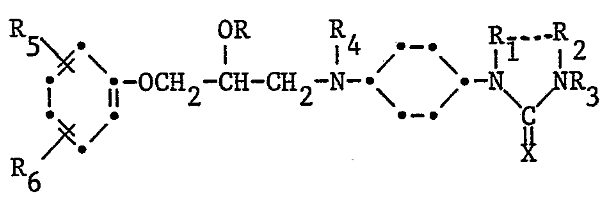

(II),

worin R Wasserstoff oder Niederalkanoyl bedeutet; jedes der Symbole $R_1$ und $R_2$ für Wasserstoff steht, oder $R_1$ und $R_2$ zusammen unverzweigtes Alkylen mit 2 bis 4 Kohlenstoffatomen bedeuten; $R_3$ und $R_4$ unabhängig voneinander Wasserstoff oder Niederalkyl bedeuten; $R_5$ und $R_6$ unabhängig voneinander Wasserstoff, niederes (Alkyl, Alkenyl oder Alkinyl), niederes (Alkoxy, Alkenyloxy oder Alkinyloxy), Hydroxy, Cyano, Halogen, Amino, niederes (Mono- oder Dialkylamino, Alkanoylamino oder Alkylsulfonylamino), niederes (Alkylthio, Alkylsulfinyl oder Alkylsulfonyl), Morpholino, 1- oder 2-Pyrrolyl, Phenyl, 5- bis 7-gliedriges Cycloalkyl, Niederalkanoyl, Niederalkoxycarbonyl, Carbamoyl oder Sulfamoyl bedeuten; $R_6$ auch Niederalkyl oder Niederalkoxy bedeutet, welche Reste durch einen Substituenten der Gruppe Cycloalkyl mit 3 bis 6 Kohlenstoffatomen, Phenyl, Niederalkoxy, Hydroxy, Niederalkoxycarbonylamino, Niederalkyl(thio, sulfinyl oder sulfonyl), oder durch Carbamoyl substituiert sein können; X O oder S bedeutet; und ihre Salze, insbesondere ihre therapeutisch verwendbaren Salze.

Bevorzugt sind weiter Verbindungen der Formel II, worin jedes der Symbole $R_3$ und $R_4$ Wasserstoff bedeutet; $R_5$ für Wasserstoff steht; $R_6$ Niederalkyl, Niederalkenyl, Niederalkinyl, Niederalkyl-(thio, sulfinyl oder sulfonyl), Niederalkoxy, Niederalkenyloxy, Niederalkinyloxy, Cyan, Niederalkoxycarbonyl, Carbamoyl, Niederalkanoylamino, Morpholino, Pyrrolyl oder 5- bis 7-gliedriges Cycloalkyl bedeutet; $R_6$ auch für Niederalkyl oder Niederalkoxy steht, welche Reste durch Cycloalkyl mit 3 bis 5 Kohlenstoffatomen, durch Phenyl, durch Niederalkoxy oder durch Carbamoyl substituiert sind; X O bedeutet und ihre Salze, insbesondere ihre therapeutisch verwendbaren Salze.

Von besonderem Interesse sind Verbindungen der Formel III

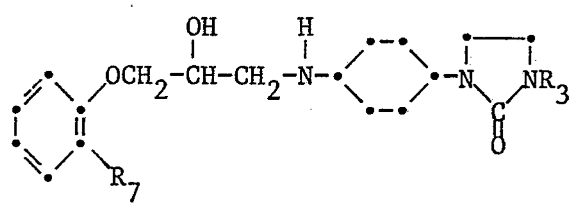

(III) ,

worin $R_3$ Wasserstoff oder Niederalkyl bedeutet; $R_7$ für Cyan, Niederalkoxycarbonyl, Pyrrolyl, Morpholino, Alkenyloxy mit 3 bis 6 Kohlenstoffatomen, Alkinyloxy mit 3 bis 6 Kohlenstoffatomen oder Alkoxy mit 1 bis 3 Kohlenstoffatomen, welches durch Cyclopropyl substituiert ist, steht; und ihre Salze, insbesondere ihre therapeutisch verwendbaren Salze.

Die Verbindungen der Formeln I, II und III können in zwei isomeren Formen vorliegen, bei welchen die beiden Reste am Cyclohexanring (d.h. die beiden 1,4-Cyclohexylen-Substituenten), entweder cis- oder transständig zueinander sind. Ferner kann das asymmetrische Kohlenstoffatom, das den OR-Rest trägt, welcher Hydroxy, Alkanoyloxy oder Aroyloxy sein kann, in erfindungsgemässen Verbindungen entweder S- oder R-Konfiguration besitzen. Daher können die Verbindungen gemäss der Erfindung in Form von Stereoisomeren auftreten, z.B. als geometrische Isomeren, Racemate, reine Enantiomeren oder Mischungen aus diesen, die alle im Rahmen der vorliegenden Erfindung liegen.

Bevorzugt sind erfindungsgemässe Verbindungen, zum Beispiel solche der Formeln I, II oder III, worin die beiden 1,4-Cyclohexylen-Substituenten zueinander cis-ständig sind.

Ferner bevorzugt sind die Enantiomere, bei welchen das die Hydroxy-, Alkaloyloxy- oder Aroyloxygruppe tragende Kohlenstoffatom S-Konfiguration besitzt.

Von ganz besonderem Interesse sind Verbindungen der Formel III, worin $R_3$ Wasserstoff und $R_7$ Allyloxy, Propargyloxy oder Cyclopropylmethoxy bedeutet; die Stereoisomeren und Enantiomeren davon; und ihre Salze, insbesondere ihre therapeutisch verwendbaren Salze. Besonders bevorzugt sind dabei die cis-Stereoisomeren und S-Enantiomeren.

Die zur Definition der erfindungsgemässen Verbindungen verwendeten allgemeinen Begriffe haben im Rahmen der vorliegenden Erfindung die folgende Bedeutung.

Ein aromatischer Rest bedeutet vorzugsweise Phenyl, Naphthyl, Pyridyl, Chinolyl, Isochinolyl, Indolyl oder 1,2,5-Thiadiazolyl. Ein partiell hydrierter bicyclischer, carbocyclischer oder heterocyclischer aromatischer Rest ist vorzugsweise Tetrahydronaphthyl, Tetrahydrochinolyl oder Tetrahydroisochinolyl. Die gegebenenfalls am Phenyl- oder am Phenylen-Teil der obengenannten bicyclischen Reste sitzenden Substituenten sind vorzugsweise Niederalkoxy, z.B. Methoxy; Niederalkyl, z.B. Methyl; Halogen, z.B. Chlor; Hydroxy; oder Trifluormethyl. Die gegebenenfalls am Heterocyclus oder am partiell hydrierten Ring der bicyclischen Reste sitzenden Substituenten umfassen vorzugsweise Niederalkoxy, Niederalkyl, Halogen, Hydroxy, Cyan, Carbamoyl, substituiertes Amino, (Alkylen, Oxaalkylen oder Thiaalkylen)-imino, z.B. Morpholino, und Oxo im Falle der obengenannten partiell hydrierten bicyclischen Reste.

Ein Alkenylrest, als solcher oder wie beispielsweise in Alkenyloxy vorhanden, bedeutet unverzweigtes oder verzweigtes Alkenyl, vorzugsweise mit bis zu 7 Kohlenstoffatomen, z.B. 2-Propenyl (Allyl), 2-Butenyl, 2-Methyl-2-propenyl oder 2-Methyl-2-butenyl.

Ein Alkinylrest, als solcher oder wie beispielsweise in Alkinyloxy vorhanden, bedeutet unverzweigtes oder verzweigtes Alkinyl, vorzugsweise mit bis zu 7 Kohlenstoffatomen, z.B. 2-Propinyl (Propargyl), 2-Butinyl oder 2-Pentinyl.

Der Ausdruck "nieder" definiert in den oben oder nachfolgend genannten organischen Resten oder Verbindungen solche mit höchstens 7, vorzugsweise 4, insbesondere 1 oder 2 Kohlenstoffatomen.

BAD ORIGINAL

Eine Niederalkylgruppe enthält vorzugsweise 1 bis 4 Kohlenstoffatome und bedeutet zum Beispiel Aethyl, Propyl, Butyl oder insbesondere Methyl.

Eine Niederalkoxycarbonylgruppe enthält vorzugsweise 1 bis 4 Kohlenstoffatome im Alkoxyteil und bedeutet zum Beispiel Methoxycarbonyl, Propoxycarbonyl, Isopropoxycarbonyl oder insbesondere Aethoxycarbonyl.

Eine niedere Alkylengruppe kann verzweigt oder unverzweigt sein, enthält vorzugsweise 1 bis 7 Kohlenstoffatome und bedeutet insbesondere 1,2-Aethylen, 1,3-Propylen, 1,4-Butylen oder 1,5-Pentylen.

Cycloalkyl ist vorzugsweise Cyclopropyl, Cyclopentyl, Cyclohexyl oder Cycloheptyl. Oxaalkylenimino ist vorzugsweise Morpholino; Thiaalkylenimino bedeutet vorzugsweise Thiomorpholino; Niederalkylenimino bedeutet vorzugsweise Pyrrolidino oder Piperidino.

Aroyl ist vorzugsweise Benzoyl; mit 1 bis 3 Niederalkyl-, Niederalkoxy-, Halogen- oder Trifluormethylresten substituiertes Benzoyl oder Heteroaroyl, z.B. Nicotinoyl.

Niederalkanoyl ist vorzugsweise Acetyl, Propionyl oder Butyryl.

Niederalkoxy ist vorzugsweise Aethoxy, Propoxy, Isopropoxy oder insbesondere Methoxy.

Niederalkylthio ist vorzugsweise Aethylthio, Propylthio oder insbesondere Methylthio.

Niederalkylsulfinyl ist vorzugsweise Aethylsulfinyl, Propylsulfinyl oder insbesondere Methylsulfinyl.

Niederalkylsulfonyl ist vorzugsweise Aethylsulfonyl, Propylsulfonyl oder insbesondere Methylsulfonyl.

Niederalkoxycarbonylamino ist vorzugsweise Aethoxycarbonylamino oder Methoxycarbonylamino.

Halogen ist vorzugsweise Fluor oder Chlor, kann aber ebenso Brom oder Jod sein.

Alkylamino ist vorzugsweise Mono- oder Di-(methyl, äthyl, propyl)-amino.

Alkylcarbamoyl ist vorzugsweise Mono- oder Di-N-(methyl, äthyl, propyl)-carbamoyl.

Alkylsulfamoyl ist vorzugsweise Mono- oder Di-N-(methyl, äthyl, propyl)-sulfamoyl.

Alkanoylamino ist vorzugsweise Acetylamino oder Propionylamino.

Alkylsulfonylamino ist vorzugsweise Methylsulfonylamino oder Aethylsulfonylamino.

Die genannten Verbindungen der Formel I bilden Säureadditionssalze, die vorzugsweise solche von therapeutisch verwendbaren anorganischen oder organischen Säuren sind, wie beispielsweise von starken Mineralsäuren, zum Beispiel Halogenwasserstoffsäuren, z.B. Salzsäure oder Bromwasserstoffsäure; Schwefel-, Phosphor-, Salpeter- oder Perchlorsäure; aliphatischen oder aromatischen Carbonsäuren oder Sulfonsäuren, z.B. Ameisen-, Essig-, Propion-, Bernstein-, Glykol-, Milch-, Aepfel-, Wein-, Glucon-, Zitronen-, Malein-, Fumar-, Pyruvin-, Phenylessig-, Benzoe-, 4-Aminobenzoe-, Anthranil-, 4-Hydroxybenzoe-, Salicyl-, 4-Aminosalicyl-, Pamoe-, Nikotin-; Methansulfon-, Aethansulfon-, Hydroxyäthansulfon-, Benzolsulfon-, p-Toluolsulfon-, Naphthalinsulfon-, Sulfanil-, Cyclohexylsulfaminsäure; oder von Ascorbinsäure.

In Ergänzung zu den obengenannten pharmazeutisch verwendbaren Salze sind auch alle "prodrug"-Derivate, zum Beispiel therapeutisch verwendbare Ester der erfindungsgemässen Alkohole (Verbindungen der Formel I, worin R Wasserstoff ist), die sich durch Solvolyse oder unter physiologischen Bedingungen zu den genannten Alkoholen umwandeln lassen, Gegenstand der vorliegenden Erfindung.

Solche Ester sind vorzugsweise Niederalkanoylester, beispielsweise der Acetyl- oder Isobutyrylester; Aroylester, z.B. der Benzoyl- oder Nikotinoylester; Carbamoylester (Carbamate), z.B. der N-Aethylcarbamoyl- oder N-Methylcarbamoylester.

Die Verbindungen der vorliegenden Erfindung zeigen wertvolle pharmakologische Eigenschaften, in erster Linie kardiovaskuläre, zum Beispiel antihypertensive und herzbeeinflussende Wirkungen, welche sie unter anderem aufgrund ihrer Hemmwirkung auf β-adrenergische Rezeptoren hervorrufen. Diese Zusammenwirkung wird auf die starke Affinität der erfindungsgemässen Verbindungen zu β-adrenergischen Rezeptoren zurückgeführt. Bei Verbindungen dieser Erfindung, die keine oder nur geringe an sich β-stimulierende Aktivität aufweisen, führt diese β-Rezeptor-andrenergische Rezeptoraffinität zu einer starken adrenergischen Hemmwirkung. Zusätzlich weisen die erfindungsgemässen Verbindungen α-adrenergische Blockierungseigenschaften auf, die ebenfalls z.B. zu ihrer antihypertensiven Wirkung beitragen können, wobei sie weitgehend keine Neigung haben, orthostatische Hypotension zu verursachen.

Die obengenannten pharmakologischen Eigenschaften können durch in vitro oder in vivo Tierversuche, vorzugsweise an Säugetieren, wie Ratten, Katzen, Hunden oder ihren isolierten Organen, als Testobjekte nachgewiesen werden. Die Tiere können entweder normotensiv oder hypertensiv, z.B. genetisch hypertensive Ratten, oder renal hypertensive Ratten oder Hunde, und Hunde, denen Natrium entzogen worden ist, sein. Die genannten Verbindungen können ihnen enteral oder parenteral, vorzugsweise oral oder intravenös, z.B. durch Gelatine-Kapseln oder in

Form von Stärke enthaltenden Suspensionen bzw. wässerigen Lösungen, verabreicht werden. Die verwendete Dosis kann in einem Bereich von ungefähr zwischen 0,01 und 100 mg/kg/Tag, vorzugsweise ungefährt 0,05 bis 50 mg/kg/Tag, insbesondere ungefähr 0,1 bis 30 mg/kg/Tag liegen.

Die in vivo blutdrucksenkende Wirkung wird entweder direkt mit einem Katheter, der z.B. in die fumurale Arterie eines Hundes eingeführt ist, oder indirekt durch Sphygmomanometrie am Rattenschwanz, und einem Uebertragungsinstrument registriert. Der Bludruck wird vor und nach der Verabreichung des Wirkstoffes in mmHg bestimmt. So sind z.B. die repräsentativen Verbindungen dieser Erfindung, welche in den Beispielen illustriert werden, in hypertensiven Ratten oder Hunden bereits bei einer Verabreichung in niedrigen oralen Dosen von 10 mg/kg/Tag oder darunter, sehr wirksam.

So kann die antihypertensive Wirkung und die Reduktion der Herzfrequenz bei spontan hypertensiven Ratten nachgewiesen werden, indem man indirekt durch Sphygmomanometrie am Rattenschwanz den systolischen Druck misst. Wache Ratten werden einzeln in Käfige gesperrt, welche sich in einem angenehm warmen Raum befinden. Nachdem Kontrollwerte für Blutdruck und Herzfrequenz gemessen worden sind, werden die Testverbindungen oral einmal täglich an 2 oder 4 aufeinanderfolgenden Tagen verabreicht. Der Blutdruck wird gewöhnlich 24 Stunden nach der ersten Dosis und dann jeweils 2,0, 4,0 und 24 Stunden nach jeder täglichen Dosis bestimmt, und die Wirkung mit der bei solchen Ratten verglichen, die mit dem Trägermaterial behandelt worden sind.

Als typische Vertreter der erfindungsgemässen Verbindungen weisen die Verbindungen der Beispiele 1, 2 und 3m eine deutliche antihypertensive und bradycardische (d.h. die Herzfrequenz herabsetzende) Wirkung auf, wenn sie spontan hypertensiven Ratten in niedrigen Dosen von 10 mg/kg oder darunter verabreicht werden.

Die antihypertensive und bradycardische Wirkung wird ebenso an wachen, chronisch hypertensiven Hunden untersucht; die Hypertension wird dabei durch eine Schwächung der Nierenfunktion erzeugt. Der arterielle Druck wird durch direkte perkutane Punktion der femuralen Arterie, unter Verwendung einer Nadel, die an ein Uebertragungsinstrument angeschlossen ist, registriert. Nach der Aufnahme von Kontrolldaten werden die Hunde oral einmal täglich, 4 Tage lang, mit Kapseln, welche die Testverbindung enthalten, behandelt. Der Blutdruck und die Herzfrequenz werden jeweils 1,5, 3, 6 und 24 Stunden nach der täglichen Dosis bestimmt und die Ergebnisse mit den vor der Behandlung erhaltenen Kontrollwerten verglichen.

Beispielsweise können die Verbindungen der Beispiele 1 und 3m, bei Verabreichung einer oralen Dosis von 10 mg/kg, bei renal hypertensiven Hunden eine ununterbrochene Verringerung des mittleren arteriellen Druckes bewirken und die Herzfrequenz herabsetzen.

Die β-adrenergische Rezeptorbindungseigenschaften, welche β-adrenergische regulierende, z.B. blockierende Wirkung der genannten neuen Verbindungen anzeigen, werden in einem Test der β-Rezeptorbindung in vitro nach einer Modifikation des Verfahrens bestimmt, wie es in Life Sciences 17, 993 (1975) beschrieben ist.

$^3$H-Dihydroalprenolol wird spezifisch von Gehirnmembran-Präparaten gebunden und diese Wirkung wird durch bekannte Agonisten und Antagonisten von β-adrenergischen Rezeptoren gehemmt.

Beim Bindungstest werden 2 ml-Proben der Suspension eines Membranpräparates (welches etwa 20 mg Kalbshirn-Caudaten entspricht) in eisgekühlte Reagenzgläser gegeben, welche $^3$H-Dihydroalprenolol mit oder ohne Testverbindung, frisch gelöst in 0,1% Ascorbinsäure, enthalten. Die Endkonzentration von $^3$H-Dihydroalprenolol ist 1,0 nM. Die Testverbindungen werden innerhalb einer weiten Konzentrationsreihe geprüft. Die Reagenzgläser werden 10 Minuten bei 37°C inkubiert, und die

Suspensionen dann sofort unter Vakuum durch Fiberglasfilter filtriert. Die Filter werden mit 15 ml kalter 50 mM Tris-HCl (pH 7,9 bei 25°C) gewaschen, zusammen mit 12 ml Szintillationslösung in Szintillationsampullen gegeben, zunächst 90 Minuten geschüttelt und dann mit dem Zählrohr auf Radioaktivität untersucht.

Die $IC_{50}$-Werte (welche die Konzentration der Testverbindungen angeben, die nötig ist, um die spezifische Bindung der 1,0 nM $^3$H-Dihydrcalprenolol-Lösung um 50% zu verringern) werden graphisch bestimmt.

Die erfindungsgemässen Verbindungen hemmen die β-Rezeptorbildung des Dihydroalprenolols mit $IC_{50}$-Werten von nur 5 nM (5 x $10^{-9}$M) oder darunter. Beispielsweise weisen die Verbindungen der Beispiele 1, 2a, 3b und 3c $IC_{50}$-Werte von etwa 80, 5, 50 bzw. 4 nM auf.

Die antagonistische Wirkung der erfindungsgemässen Verbindungen auf β-adrenergische Rezeptoren kann man in vitro durch den Antagonismus der Epinephrin-Aktivierung eines Adenylatcyclase-Präparates, das vom Kleinhirn eines Meerschweinchens stammt, zeigen. [J. Neurochemistry, 22. 1031 (1974)]. Beispielsweise zeigen die erfindungsgemässen Verbindungen der Beispiele 1, 2a und 3b einen $IC_{50}$-Wert von etwa 3-5 nM (3-5 x $10^{-9}$M), was eine starke β-adrenergische Blockade beweist.

Die α-adrenergischen Rezeptorbindungseigenschaften, welche die α-Rezeptorblockierungseigenschaften anzeigen, werden z.B. in vitro durch die Hemmung der Bindung vom bekannten α-Blocker Prazocin ($^3$H-Prazocin) an ein synaptosonales Membranpräparat aus dem Rattenvorderhirn nachgewiesen [Naunyn-Schmiedebergs Arch. Pharmacol. 308, 223 (1979)].

Beispielsweise wirken die Verbindungen der Beispiele 1, 2a, 3b und 3m im oben geschilderten α-Rezeptorbindungstest mit IC-Werten, die bei 1 µM (1 x $10^{-6}$M) liegen.

Die Anticlaucoma-Wirkung der erfindungsgemässen Verbindungen ist an der intraokularen Druckverminderung abzulesen, die an Säugetieren, z.B. bei normotensiven Kaninchen, im wesentlichen nach der von D.E. Potter und J.M. Rowland in Experimental Eye Research 27, 615-625, (1978) beschriebenen Methodik nachgewiesen werden kann. Die intraokulare Druckverminderung wird wie folgt bestimmt: 2 x 50 µl einer Lösung der Testverbindung in sterilisiertem Wasser von unterschiedlicher Konzentration werden bei männlichen, neuseeländischen Albinokaninchen, die 3-4 kg wiegen, jeweils auf ein Auge appliziert, und 2 x 50 µl des Trägermaterials werden zur Kontrolle auf das kontralaterale Auge appliziert. Der intraokulare Druck (in mm Hg) in jedem Auge wird direkt vor der Behandlung tonometrisch gemessen, und dann 1,2 bzw. 3 Stunden nach der Verabreichung. Es wird der Unterschied des intraokularen Druckes zwischen behandeltem Auge und Kontrollauge bestimmt.

Beispielsweise zeigen die erfindungsgemässen Verbindungen der Beispiele 1 und 2a, bei einer Konzentration von etwa 20 bzw. 6,4 nM eine deutliche Verminderung des intraokularen Druckes bei Kaninchen.

Dementsprechend können die Verbindungen der vorliegenden Erfindung bei Säugetieren als α- und β-adrenergische Blocker eingesetzt werden, ferner als kardiovaskuläre Wirkstoffe, insbesondere als antihypertensive und die Herzfrequenz herabsetzende Mittel, zum Beispiel in der Behandlung, Handhabung und Vorbeugung kardiovaskulärer Zustände wie Hypertonie oder Herzstörungen wie Myokardinfarkten, Herzrhythmusstörungen oder Angina pectoris, und/oder anderer Zustände, die unter anderem auf eine β-adrenergische Blockade ansprechen, wie Erregbarkeit, Beklemmungen, Glaukoma oder Migräne. Sie können auch als Zwischenprodukte zur Herstellung von anderen wertvollen Produkten, insbesondere von pharmakologisch wirksamen Präparaten, eingesetzt werden.

Die vorliegende Erfindung betrifft auch Methoden zur Herstellung der Verbindungen der Formel I. Wenn im folgenden nicht anders definiert, haben Ar, R, $R_1$ bis $R_4$ und X die gleiche Bedeutung wie oben unter

der Formel I angegeben.

Die Verbindungen der vorliegenden Erfindung werden nach an sich bekannten Methoden hergestellt, indem man

a) eine Verbindung der Formel IV

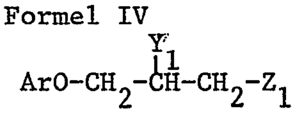

(IV)

mit einer Verbindung der Formel V

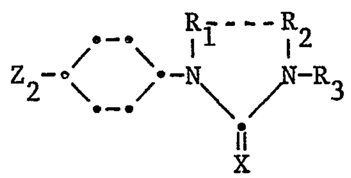

(V)

kondensiert, in welchen einer der Reste $Z_1$ und $Z_2$ eine raktionsfähige veresterte Hydroxygruppe und der andere die Gruppe $NHR_4$ bedeutet, und $Y_1$ für Hydroxy, Alkanoyloxy oder Aroyloxy steht; oder in welchen $Y_1$ und $Z_1$ zusammen eine Epoxygruppe darstellen, $Z_2$ die Aminogruppe $NHR_4$ bedeutet, und Ar, $R_1$, $R_2$, $R_3$, $R_4$ und X die oben angegebenen Bedeutungen haben; und wenn erwünscht, eine erhaltene Verbindung der Formel I in eine andere Verbindung der Formel I umwandelt; und/oder, wenn erwünscht, eine erhaltene freie Verbindung in ein Salz oder ein erhaltenes Salz in eine freie Verbindung oder in ein anderes Salz überführt; und/oder, wenn erwünscht, ein erhaltenes Isomerengemisch in die Isomeren auftrennt oder ein erhaltenes Racemat in die beiden Enantiomeren spaltet.

Eine reaktionsfähige veresterte Hydroxygruppe $Z_1$ oder $Z_2$ ist eine Hydroxygruppe, die durch eine starke Säure verestert ist, insbesondere durch eine starke anorganische Säure wie etwa eine Halogenwasserstoffsäure, z.B. Salzsäure, Brom- oder Jodwasserstoffsäure, oder Schwefelsäure, oder durch eine starke organische Säure, insbesondere durch eine starke organische Sulfonsäure wie etwa eine aliphatische oder aromatische Sulfonsäure, z.B- Methansulfonsäure, 4-Methylphenylsulfonsäure oder 4-Bromphenylsulfonsäure.

Die genannte reaktionsfähige veresterte Hydroxygruppe ist insbesondere Halogen, z.B. Chlor, Brom oder Jod, oder aliphatisch oder aromatisch substituiertes Sulfonyloxy, z.B. Phenylsulfonyloxy oder 4-Methylphenylsulfonyloxy (Tosyloxy).

Die obengenannte Reaktion wird in an sich bekannter Weise durchgeführt, gewöhnlich in Gegenwart eines Lösungsmittels oder einer Mischung aus mehreren Lösungsmitteln und, falls erforderlich, unter Kühlung oder unter Erwärmung, beispielsweise in einem Temperaturintervall von etwa -20°C bis etwa 150°C, in einem offenen oder geschlossenen Gefäss und/oder in einem Inertgas, beispielsweise in einer Stickstoffatmosphäre. Verwendet man einen Ausgangsstoff, der eine reaktionsfähige veresterte Hydroxygruppe besitzt, wird die Reaktion vorteilhaft unter basischen Bedingungen durchgeführt, etwa in Gegenwart einer anorganischen Base, z.B. eines Alkalimetall- oder Erdalkalimetallcarbonates oder -hydroxids oder in Gegenwart einer organischen Base wie etwa eines Alkalimetallniederalkanolats und/oder in einem Ueberschuss des basischen Reaktionsmittels.

Insbesondere werden Verbindungen der Formel I hergestellt, indem man ein Oxiran der Formel VI

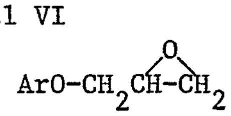

(VI)

mit einem Amin der Formel VII

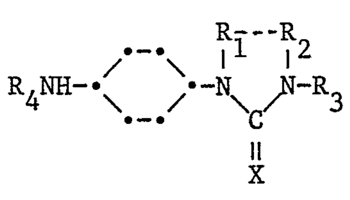

(VII)

oder einem seiner Stereoisomeren kondensiert, worin $R_1$ und $R_2$ unabhängig voneinander Wasserstoff oder Niederalkyl bedeuten; oder $R_1$ und $R_2$ zusammen für Alkylen mit 2 bis 7 Kohlenstoffatomen stehen, welches die beiden Stickstoffatome, an denen es anknüpft, durch 2 bis 4 Kohlenstoffatome trennt; $R_3$ und $R_4$ unabhängig voneinander Wasserstoff oder

Niederalkyl bedeuten, und X Oxo oder Thio ist.

Bevorzugte Zwischenprodukte der Formel VII sind die Verbindungen der Formel VIII

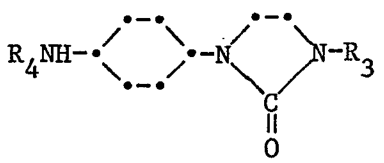

(VIII)

oder ihre Stereoisomeren, vorteilhaft die cis-Isomeren, in welchen $R_3$ und $R_4$ Wasserstoff oder Niederalkyl bedeuten.

Die Ausgangsstoffe der Formel IV (und z.B. VI) sind in der Literatur bekannt oder können nach an sich bekannten Methoden hergestellt werden, z.B. so wie in den Beispielen dieser Erfindung veranschaulicht. Zum Beispiel werden racemische oder optisch aktive Ausgangsverbindungen der Formel IV dadurch hergestellt, dass man zunächst Verbindungen der Formel ArOH oder ihre Alkalimetall-Derivate mit z.B. racemischem oder optisch aktivem Epichlorhydrin oder Benzyl-2,3-epoxypropyläther kondensiert und die erhaltenen Produkte hydrogenolysiert, acyliert und/oder cyclisiert.

Die Ausgangsstoffe der Formeln VII und VIII (Verbindungen der Formel V mit $Z_2$ = $NHR_4$), werden bevorzugt dadaurch hergestellt, dass man z.B. Verbindungen der Formel IX

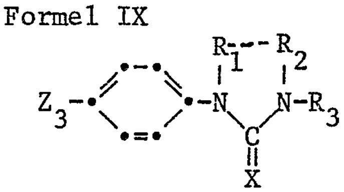

(IX)

hydriert, worin $Z_3$ $R_4NH$, Nitro oder Acyl-$NHR_4$ darstellt; $R_1$, $R_2$, $R_3$, $R_4$ und X die oben definierten Bedeutungen haben; Acyl-$NHR_4$ acyliertes $NHR_4$ ist, z.B.(Alkoxycarbonyl- oder Aralkoxycarbonyl-, z.B. tert.-Butyloxycarbonyl-, Carbobenzoxy-; Niederalkanoyl-, z.B. Acetyl- oder Trifluoracetyl)-substituiertes $NHR_4$.

Die Hydrierung von Verbindungen der Formel IX wird nach an sich bekannten Methoden durchgeführt, vorzugsweise mit katalytisch aktiviertem

BAD ORIGINAL

Wasserstoff, wie etwa Wasserstoff in Gegenwart z.B. eines Platin-, Palladium-, Nickel-, vorzugsweise eines Rhodiumkatalysators, in einem Lösungsmittel wie z.B. Wasser, Essigsäure oder Aethanol, bei Raumtemperatur oder bei erhöhter Temperatur, und bei normalem oder erhöhtem Druck, z.B. bei einem Druck von 3 Atmosphären.

Die erhaltenen Verbindungen, die man durch Hydrierung der Verbindungen der Formel IX, worin $Z_2$ Acyl-$NHR_4$ bedeutet, erhält, werden zu Verbindungen der Formel VII (das sind Verbindungen der Formel V mit $Z_2$ = $NHR_4$) umgewandelt, indem man die Acyl-Schutzgruppe abspaltet, zum Beispiel durch Hydrolyse mit einer wässerigen Base wie z.B. Natronlauge oder einer wässerigen Säure wie z.B. Salzsäure, oder durch Hydrierung mit Wasserstoff in Gegenwart eines Katalysators wie z.B. Palladium.

Weiterhin können die entsprechenden Zwischenprodukte der Formeln VII und VIII, worin $R_4$ Wasserstoff bedeutet, in Zwischenprodukte der Formeln VII oder VIII, worin $R_4$ Niederalkyl ist, umgewandelt werden, zum Beispiel durch Alkylierung mit einem reaktionsfähigen veresterten Niederalkanol wie etwa einem Niederalkylhalogenid oder unter den Bedingungen der reduktiven Alkylierung, z.B. mit Formaldehyd in Gegenwart von Ameisensäure, oder mit Aceton in Gegenwart von Natriumcyanborhydrid.

Die Zwischenprodukte der Formeln V, VII und VIII können als cis- oder trans-Isomeren oder als cis/trans-Gemische erhalten werden. Wenn ein cis/trans-Isomerengemisch erhalten wird, können die Isomeren nach Methoden, die dem Stand der Technik entsprechen, getrennt werden, z.B. durch Chromatographie oder Kristallisation. Vorteilhaft werden die cis-Isomeren der Amine der Formeln VII und VIII isoliert, die zu den bevorzugten Verbindungen der vorliegenden Erfindung führen, entweder als freie Basen oder als Säureadditionssalze.

Ein weiteres Verfahren zur Herstellung von Verbindungen der Formel I

besteht darin, dass man

b) eine Verbindung der Formel ArOH, vorzugsweise in Form eines Metallsalzes, z.B. eines Alkalimetall-Derivates, mit einer Verbindung der Formel X

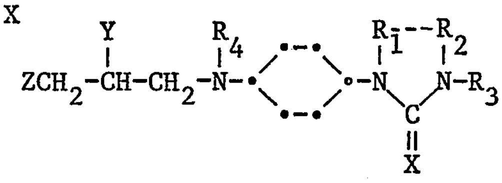

(X) ,

worin Z reaktionsfähiges verestertes Hydroxy und Y OR bedeutet; oder Z und Y zusammen eine Epoxygruppe bilden; und Ar, R, $R_1$, $R_2$, $R_3$, $R_4$ und X die oben definierten Bedeutungen haben und worin die Amino- und Hydroxygruppen sowie andere funktionelle Gruppen gegebenenfalls in geschützter Form vorliegen, kondensiert oder mit einer Verbindung der Formel XI

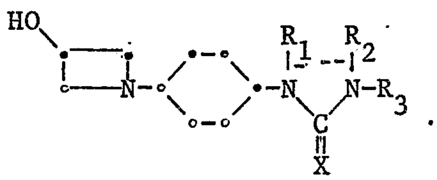

(XI) ,

worin $R_1$, $R_2$, $R_3$ und X die oben definierten Bedeutungen besitzen und worin Hydroxy gegebenenfalls in geschützter Form vorliegen kann, kondensiert und, wenn erwünscht, eine erhaltene Verbindung der Formel I in eine andere erfindungsgemässe Verbindung umwandelt.

Die obigen Kondensationen können unter an sich bekannten Bedingungen durchgeführt werden, vorteilhaft in einem inerten Lösungsmittel, bei Raumtemperatur oder erhöhter Temperatur und vorzugsweise in einer Inertgasatmosphäre.

Zwischenprodukte der Formel XI können durch Cyclisierung einer Verbindung der Formel X, worin Z reaktionsfähiges verestertes Hydroxy, Y Hydroxy und $R_4$ Wasserstoff bedeutet, erhalten werden. Zwischenprodukte der Formel X können durch Kondensation der Amine der Formel VII mit z.B. Epichlorhydrin, hergestellt werden.

Eine weitere Möglichkeit zur Herstellung von Verbindungen der Formel I besteht darin, dass man

c) eine Verbindung der Formel XII

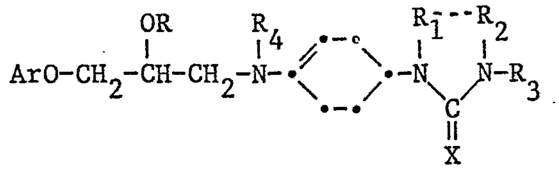

(XII)

oder, wenn $R_4$ Wasserstoff bedeutet, eines ihrer Tautomeren, reduziert, wobei Ar, R, $R_1$, $R_2$, $R_3$, $R_4$ und X die oben angegebenen Bedeutungen haben, und, wenn erwünscht, eine erhaltene Verbindung der Formel I in eine andere Verbindung der Erfindung umwandelt.

Die Reduktion kann entweder durch katalytische Hydrierung erfolgen, zum Beispiel mit Wasserstoff in Gegenwart von Raney-Nickel als Katalysator, oder durch ein Reduktionsmittel wie etwa Natriumcyanborhydrid, wobei die Reaktion nach an sich bekannten Methoden durchgeführt wird, zum Beispiel in einem Lösungsmittel wie Aethanol, vorzugsweise bei Raumtemperatur oder leicht erhöhter Temperatur.

Zwischenprodukte der Formel XII, worin R Wasserstoff bedeutet, können als Isomeren, wie in Formel XIIa

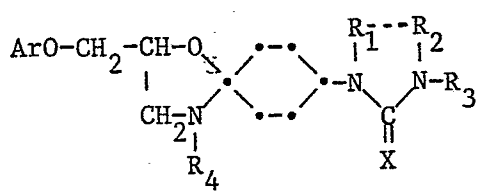

(XIIa)

gezeigt, vorliegen.

Die Zwischenprodukte der Formeln XII und XIIa können, z.B. in situ, durch Kondensation eines Amins der Formel XIII

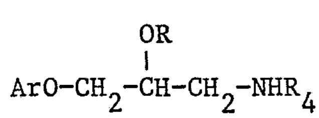

(XIII)

mit einen Keton der Formel XIV

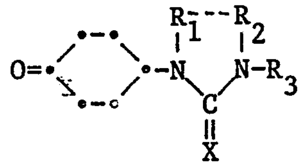 (XIV)

hergestellt werden, wobei eine solche Kondensation entweder spontan abläuft oder vorzugsweise in Gegenwart eines Dehydratisierungsmittels wie etwa Bortrifluorid, Magnesiumsulfat oder Molekularsieb.

Die Amine der Formel XIII, von denen man ausgeht, können durch Kondensation einer Verbindung der Formel VI mit $R_4NH_2$ unter den Standardbedingungen einer Aminolyse erhalten werden. Ketone der Formel XIV können dann wieder durch Oxidation von Verbindungen der Formel VII, beispielsweise mit einem Alkalimetallhypochlorit, hergestellt werden, vorzugsweise unter den Bedingungen der Phasentransferkatalyse wie in Tetrahedron Letters 1976, 1641 beschrieben.

Ferner können Verbindungen der vorliegenden Erfindung dadurch hergestellt werden, dass man

d) eine Verbindung der Formel XV

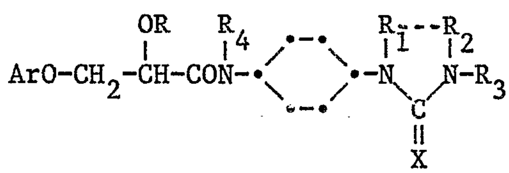 (XV)

reduziert, und, wenn erwünscht, eine erhaltene Verbindung der Formel I in eine andere Verbindung der Erfindung umwandelt.

Die Reduktion wird beispielsweise mit einfachen oder komplexen Leichtmetallhydriden wie z.B. Alan, Boran oder Lithiumaluminiumhydrid, gemäss Verfahren, die in der Technik wohlbekannt sind, durchgeführt.

Zwischenprodukte der Formel XV können wiederum durch Kondensation einer Säure der Formel XVI

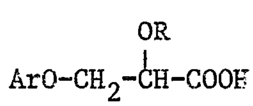 (XVI),

falls R Wasserstoff bedeutet, gegebenenfalls in geschützter Form oder eines ihrer reaktionsfähiger funktioneller Derivate, z.B. eines Säurechlorids oder eines gemischten Anhydrids, mit einem Amin der Formel VII gemäss wohlbekannten Verfahren zur Herstellung von Amiden, hergestellt werden.

Die Kondensation einer freien Carbonsäure der Formel XVI mit einem Amin der Formel VII wird vorzugsweise in Gegenwart eines Kupplungsreagens, wie etwa Dicyclohexylcarbodiimiden oder 1,1'-Carbonyldiimidazol, gemäss in der Technik wohlbekannten Verfahren durchgeführt.

Die Kondensation eines reaktionsfähigen funktionellen Derivats einer Verbindung der Formel XVI mit einem Amin der Formel VII verläuft spontan oder in Gegenwart von Basen wie etwa Kaliumcarbonat, Pyridin oder Triäthylamin, in einem inerten Lösungsmittel wie etwa Methylenchlorid, bei Raumtemperatur oder erhöhter Temperatur.

Die Säuren der Formel XVI können im wesentlichen hergestellt werden nach dem im US-Patent 3,699,097 beschriebenen Verfahren, beispielsweise durch Kondensation von ArOH mit Chlormilchsäure in wässeriger Natronlauge.

Weiterhin können die Verbindungen der vorliegenden Erfindung dadurch hergestellt werden, dass man

e) eine Verbindung der Formel XVII

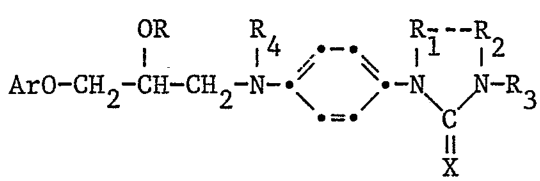

(XVII)

reduziert und, wenn erwünscht, eine Verbindung der Formel I in eine andere Verbindung der Erfindung umwandelt.

Die Reduktion wird vorteilhaft z.B. durch selektive katalytische Hydrierung mit Wasserstoff in Gegenwart eines Katalysators wie etwa

Nickel, Rhodium oder Platin, vorzugsweise im sauren Medium wie etwa Essigsäure oder Aethanol in Gegenwart einer Mineralsäure, vorgenommen. Die genannte Reduktion ist vor allem dann nützlich, wenn Ar einen Rest darstellt, der bei den Hydrierungsbedingungen relativ resistent ist.

Die Ausgangsverbindungen XVIII können durch Kondensation einer Verbindung der Formel IV mit einer Verbindung der Formel IX, worin $Z_3$ $R_4NH$ bedeutet, hergestellt werden, wobei die Bedingungen ähnlich denen gewählt werden können, wie sie für die Kondensation von Verbindungen der Formel IV mit Verbindungen der Formel V, worin $Z_2$ $NHR_4$ ist, beschrieben sind.

Ein weiteres Verfahren zur Herstellung von Verbindungen der Formel I besteht darin, dass man

f) in einer Verbindung der Formel XVIII

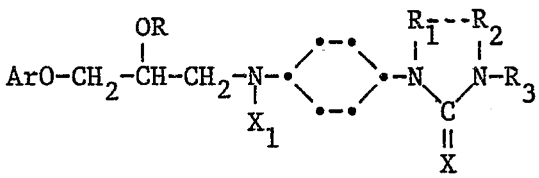

(XVIII) ,

worin $X_1$ einen abspaltbaren, durch Wasserstoff ersetzbaren Rest bedeutet, diesen durch Wasserstoff ersetzt, und, wenn erwünscht, eine erhaltene Verbindung in eine andere Verbindung der Erfindung umwandelt.

Die abspaltbare Gruppe $X_1$ kann durch Solvolyse, wie Hydrolyse, Alkoholyse oder Acidolyse, oder durch Reduktion, einschliesslich Hydrogenolyse durch Wasserstoff ersetzt werden. Eine geeignete, durch Hydrogenolyse abspaltbare Gruppe $X_1$ ist eine α-Aryl-niederalkylgruppe, vorzugsweise 1-Phenyl-niederalkylgruppe, insbesondere Benzyl. Eine Gruppe $X_1$ kann auch ein Rest sein, der durch Solvolyse, wie Hydrolyse oder Acidolyse, oder auch Reduktion abgespalten werden kann. Ein solcher Rest ist ein entsprechender Acylrest, wie der Acrylrest einer organischen Carbonsäure, wie Acetyl oder Aroyl, wie Benzoyl, ferner der Acylrest eines Halbesters der Kohlensäure, wie Niederalkoxycarbonyl, z.B. Methoxycarbonyl, Aethoxycarbonyl oder tert.-Butyloxycarbonyl, 2-Halogenniederalkoxycarbonyl, z.B. 2,2,2-Trichloräthoxycarbonyl oder 2-Jodäthoxycarbonyl, gegebenenfalls substituiertes 1-Phenylniederalkoxycarbonyl, z.B. Benzyloxycarbonyl oder Diphenylmethoxycarbonyl, oder Aroylmethoxycarbonyl, z.B. Phenacyloxycarbonyl, ferner eine gegebenenfalls substituierte 1-Polyphenylniederalkylgruppe, worin Substituenten, in erster Linie des Phenylteils, z.B. die oben gegebene Bedeutung haben, und in erster Linie Trityl darstellen.

Hydrogenolytisch abspaltbare Reste $X_1$, insbesondere gegebenenfalls substituierte 1-Phenylniederalkylgruppen, ferner auch geeignete Acylgruppen, wie gegebenenfalls substituiertes 1-Phenylniederalkoxycarbonyl, können durch Behandeln mit katalytisch aktiviertem Wasserstoff, z.B. mit Wasserstoff in Gegenwart eines Nickelkatalysators, wie Raney-Nickel, oder eines geeigneten Edelmetallkatalysators, abgespalten werden.

Hydrolytisch abspaltbare Gruppen $X_1$ wie Acylreste von organischen Carbonsäuren, z.B. Niederalkanoyl, und von Halbestern der Kohlensäure, z.B. Niederalkoxycarbonyl, ferner z.B. Tritylreste, können je nach Art solcher Reste durch Behandeln mit Wasser unter sauren oder basischen Bedingungen, z.B. in Gegenwart einer Mineralsäure, wie Chlorwasserstoff- oder Schwefelsäure, oder eines Alkalimetall- oder Erdalkalimetallhydroxids oder -carbonats oder eines Amins, wie Isopropylamin, abgespalten werden.

Acidolytisch abspaltbare Reste $X_1$ sind insbesondere gewisse Acylreste von Halbestern der Kohlensäure, wie z.B. tert.-Niederalkoxycarbonyl oder gegebenenfalls substituierte Diphenylmethoxycarbonylreste, ferner auch der tert.-Niederalkylrest; solche Reste können durch Behandeln mit geeigneten starken organischen Carbonsäuren, wie gegebenenfalls durch Halogen, insbesondere Fluor, substituierten Niederalkancarbonsäuren, in erster Linie mit Trifluoressigsäure (wenn notwendig, in Gegenwart eines aktivierenden Mittels, wie Anisol), sowie mit Ameisensäure abgespalten werden.

Unter reduktiv abspaltbaren Resten $X_1$ werden auch solche Gruppen verstanden, die beim Behandeln mit einem chemischen Reduktionsmittel (insbesondere mit einem reduzierenden Metall oder einer reduzierenden Metallverbindung) abgespalten werden. Solche Reste sind insbesondere 2-Halogenniederalkoxycarbonyl oder Arylmethoxycarbonyl, die z.B. beim Behandeln mit einem reduzierenden Schwermetall, wie Zink, oder mit einem reduzierenden Schwermetallsalz, wie einem Chrom(II)salz, z.B. -chlorid oder -acetat, üblicherweise in Gegenwart einer organischen Carbonsäure, wie Ameisensäure oder Essigsäure, und von Wasser abgespalten werden können.

Die Ausgangsstoffe der Formel XVIII können analog zu den oben beschriebenen Verfahrensvarianten hergestellt werden. So kann z.B. eine Verbindung der Formel ArOH oder ein Salz davon, mit einer Verbindung der Formel Xa

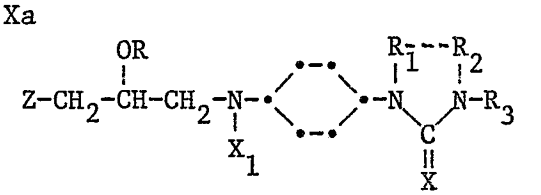
(Xa),

worin Z eine reaktionsfähige veresterte Hydroxygruppe bedeutet, und worin die funktionellen Gruppen gegebenenfalls in geschützter Form vorliegen, umgesetzt werden.

Die neuen Verbindungen der Formel I können auch dadurch erhalten werden, dass man

g) eine Schiff'sche Base der Formel XIX

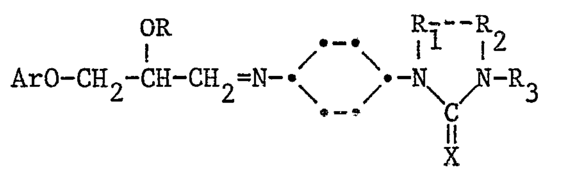
(XIX)

reduziert, und, wenn erwünscht, eine erhaltene Verbindung in eine andere Verbindung der Erfindung umwandelt.

Die Reduktion der Stickstoff-Kohlenstoff-Doppelbindung in Ausgangsstoffen der Formel XIX zur Stickstoff-Kohlenstoff-Einfachbindung kann

BAD ORIGINAL

in an sich bekannter Weise, z.B. durch Behandeln mit katalytisch aktiviertem Wasserstoff, wie Wasserstoff in Gegenwart eines geeigneten Hydrierkatalysators, z.B. eines Nickel-, Platin- oder Palladiumkatalysators, erfolgen oder man arbeitet mit einem geeigneten Hydridreduktionsmittel, wie einem Alkalimetallborhydrid, z.B. Natriumborhydrid. Bei Anwendung von Hydridreduktionsmitteln, können auch an Sauerstoff gebundene Acylreste von Carbonsäuren, wie z.B. der Essigsäure, in der Gruppe OR vorliegen und im gleichen Arbeitsgang abgespalten werden.

Ein Ausgangsstoff der Formel XIX kann in an sich bekannter Weise, gegebenenfalls in situ, d.h. unter den Reaktionsbedingungen des beschriebenen Verfahrens, hergestellt werden. So kann eine Verbindung der Formel XIXa

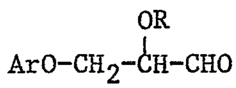

(XIXa)

mit einem Amin der Formel XIXb

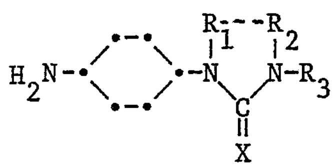

(XIXb)

zur Herstellung des Ausgangsstoffes der Formel XIX umgesetzt werden.

Ein anderes Verfahren zur Herstellung von Verbindungen der Formel I besteht darin, dass man

h) in einer Verbindung der Formel XX

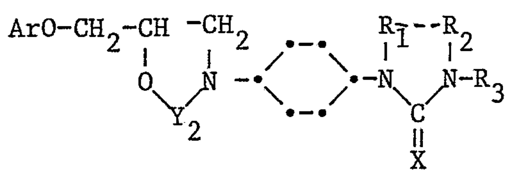

(XX)

oder in einem Salz davon, worin $Y_2$ einen zweiwertigen Rest, der durch zwei Wasserstoffatome ersetzbar ist, bedeutet, diesen Rest $Y_2$ abspaltet, und eine erhaltene Verbindung in eine andere Verbindung der Erfindung umwandelt.

Ein abspaltbarer Rest $Y_2$ ist in erster Linie eine hydrogenolytisch abspaltbare Gruppe, wie gegebenenfalls substituiertes 1-Phenyl-niederalkyliden, worin Substituenten, insbesondere des Phenylteils, z.B. Niederalkyl, wie tert.-Butyl, Hydroxy, Niederalkoxy, Halogen und/oder Nitro sein können, und insbesondere Benzyliden, sowie solvolytisch, insbesondere hydrolytisch spaltbare Gruppen, wie Niederalkyliden, z.B. Methylen oder Isopropyliden, oder Cycloalkyliden, z.B. Cyclohexyliden. Ein weiterer Rest ist der Diacylrest der Kohlensäure oder Thiokohlensäure, d.h. die Carbonyl- bzw. Thiocarbonylgruppe.

In der Form von Salzen verwendbare Ausgangsstoffe werden in erster Linie in der Form von Säureadditionssalzen, z.B. Mineralsäuren, sowie von organischen Säuren verwendet.

Die Abspaltung des Restes $Y_2$ wird analog zu den für das Verfahren f) beschriebenen Reaktionen durchgeführt.

Die Ausgangsstoffe der Formel XX können nach an sich bekannten Methoden z.B. gemäss der dem Verfahren b) analogen Umsetzung, d.h. durch Kondensation einer Verbindung der Formel ArOH mit einer Verbindung der Formel XXa

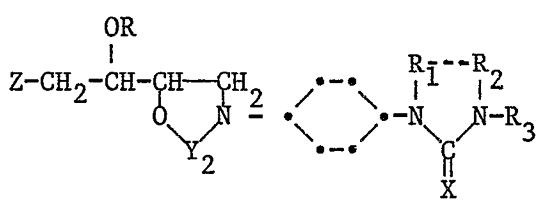

(XXa) ,

worin Z eine reaktionsfähige veresterte Hydroxygruppe bedeutet, und worin die funktionellen Gruppen gegebenenfalls in geschützter Form vorliegen, hergestellt werden.

Die neuen Verbindungen der Formel I können auch dadurch hergestellt werden, dass man

i) in einer Verbindung der Formel XXI

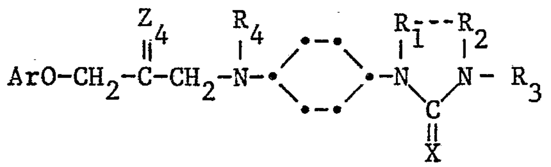 (XXI) ,

worin $Z_4$ ein Wasserstoffatom zusammen mit einer Gruppe $OZ_5$ bedeutet, worin $Z_5$ für einen abspaltbaren Rest steht; oder $Z_4$ ein Sauerstoffatom bedeutet; diesen Rest $R_4$ in ein Wasserstoffatom zusammen mit einer Hydroxygruppe umwandelt, und, wenn erwünscht, eine erhaltene Verbindung in eine andere Verbindung der Erfindung umwandelt.

Die Gruppe $OZ_5$ ist eine nach an sich bekannten Methoden geschützte Hydroxygruppe. So kann die Hydroxygruppe in der Form eines Esters, z.B. eines Acylderivates, wie eines Niederalkanoyl-, Benzyloxycarbonyl- oder Niederalkoxycarbonylesters, oder in der Form eines Aethers, z.B. als ein 2-Tetrahydropyranyl- oder Benzyläther geschützt sein. Der abspaltbare Rest $R_5$ kann durch Wasserstoff nach an sich bekannten Methoden, z.B. durch Solvolyse, insbesondere Hydrolyse mit einer Säure, oder durch Reduktion, vorzugsweise durch Hydrogenolyse, durch ein Wasserstoffatom ersetzt werden.

Das Sauerstoffatom ($Z_4$ = O) kann durch Wasserstoff zusammen mit einer Hydroxygruppe durch Reduktion nach an sich bekannten Methoden ersetzt werden. So wird die Reduktion z.B. mit katalytisch aktiviertem Wasserstoff, z.B. in Gegenwart von Raney-Nickel, Palladiumschwarz oder eines Platinkatalysators durchgeführt. Man verwendet vorzugsweise komplexe Hydride, z.B. Alkalimetallborhydride wie Natriumborhydrid, Borwasserstoffverbindungen, wie Diboren, oder Alkalimetallaluminiumhydride, wie Lithiumaluminiumhydrid, und insbesondere Alkalimetall-triniederalkylborhydride und verwandte Borverbindungen wie Kalium-tris-(sek.-butyl)-borhydrid bzw. Lithium-tris-(triamylsilyl)-borhydrid, welche in einem inerten Lösungsmittel ätherartigen Charakters, wie Dioxan, Diäthyläther oder insbesondere Tetrahydrofuran, oder im Falle der Verwendung von Natriumborhydrid auch in einem wasserfreien Alkanol oder dessen Gemisch mit Tetrahydrofuran, oder auch in wässerig-niederalkanolischer

Lösung zur Anwendung gelangen können.

Die Ausgangsstoffe können nach an sich bekannten Methoden hergestellt werden. So lassen sich z.B. Verbindungen, in welchen $Z_4$ ein Sauerstoffatom bedeutet, durch Umsetzung einer Verbindung der Formel Ar-OH oder ihrer Salze, mit einem 1,3-Dihalogenaceton und Kondensation der erhaltenen Monohalogen-aceton-Verbindung mit einem Amin der Formel XXIa

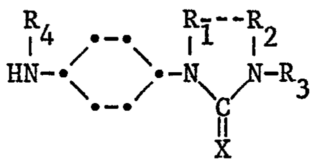

(XXIa)

herstellen.

Ausgangsstoffe, welche die geschützte Hydroxygruppe $OZ_5$ enthalten, können in analoger Weise hergestellt werden. Man verwendet dabei anstelle des 1,3-Dihalogen-acetons eine entsprechende 1,3-Dihalogenpropan-Verbindung, welche in 2-Stellung eine Gruppe $OZ_5$ aufweist.

Erfindungsgemässe Verbindungen, die nach irgendeinem der oben beschriebenen Verfahren erhalten werden, können nach konventionellen, dem Stand der Technik entsprechenden Methoden oder beispielsweise wie im folgenden illustriert, in eine andere umgewandelt werden.

Beispielsweise können Verbindungen der Formel I, worin R Alkanoyl oder Aroyl darstellt, durch Hydrolyse mit z.B. wässeriger Säure, wie etwa Salzsäure oder mit wässerigem Alkali wie etwa Lithium- oder Natriumhydroxid, in Verbindungen der Formel I umgewandelt werden, worin R Wasserstoff bedeutet.

Umgekehrt können Verbindungen der Formel I, worin R Wasserstoff ist, durch Kondensation mit einer entsprechenden Carbonsäure oder einem ihrer reaktionsfähigen Derivate, gemäss in der Technik wohlbekannten Veresterungsverfahren, vorteilhaft unter nichtbasischen Bedingungen, in Verbindungen der Formel I, worin R Alkanoyl oder Aroyl bedeutet, umgewandelt werden.

Ferner können Verbindungen der Formel I, worin $R_4$ Wasserstoff bedeutet, in eine Verbindung der Formel I, worin $R_4$ Niederalkyl bedeutet, umgewandelt werden, indem sie mit einem reaktionsfähigen veresterten Niederalkanol, z.B. mit einem Niederalkylhalogenid, umgesetzt werden, wobei die entsprechende Verbindung der Formel I vorzugsweise als Säureadditionssalz isoliert wird, oder indem man eine reduktive Alkylierung, zum Beispiel mit Formaldehyd und Ameisensäure, durchführt, wobei eine Verbindung der Formel I, worin $R_4$ Methyl ist, entsteht.

Verbindungen der Formel I, gegebenenfalls in geschützter Form, worin $R_3$ Wasserstoff bedeutet, können in Verbindungen der Formel I, worin $R_3$ Niederalkyl bedeutet, umgewandelt werden, indem sie mit einem reaktionsfähigen Derivat eines Niederalkanols, z.B. mit einem Niederalkyljodid, in Gegenwart einer starken Base wie z.B. Natriumhydrid in einem inerten Lösungsmittel wie etwa Dimethylformamid umgesetzt werden.

Ungesättigte Verbindungen, zum Beispiel solche, die einen Alkenyl- oder Alkinylrest tragen, können ebenfalls mit katalytisch aktiviertem Wasserstoff hydriert werden, wobei Verbindungen der Formel I oder Zwischenprodukte, die einen entsprechenden Alkylrest tragen, erhalten werden.

Verbindungen der Formel I, gegebenenfalls in geschützter Form, welche im Rest Ar eine Cyangruppe haben, können in die entsprechenden Carbamoylverbindungen, z.B. in einem basischen oder vorzugsweise in einem sauren Medium, insbesondere in Gegenwart einer starken Mineralsäure, z.B. konz. Chlorwasserstoffsäure, umgewandelt werden.

Enthält eine Verbindung der Formel I eine geschützte Hydroxy-niederalkylgruppe oder eine geschützte Hydroxygruppe, so kann die Schutzgruppe z.B. durch Solvolyse, wie Hydrolyse, Alkoholyse oder Acidolyse, oder durch Reduktion inklusive Hydrogenolyse, wie bereits oben beschrieben, durch Wasserstoff ersetzt werden.

In Verbindungen der Formel I, welche im Rest Ar eine Aminogruppe enthalten, kann die Aminogruppe in Niederalkylsulfonylamino durch Umsetzung mit einer entsprechenden Niederalkansulfonsäure, z.B. Methansulfonsäure, gegebenenfalls in Gegenwart eines geeigneten Kondensationsmittels, z.B. N,N'-Dicyclohexylcarbodiimids, umgewandelt werden. Anstelle der freien Säure kann auch ein reaktionsfähiges Derivat davon, z.B. ein Halogenid, wie Chlorid oder Bromid, oder ein Säureanhydrid, eingesetzt werden. Die weiteren reaktionsfähigen Substituenten eines Produktes der Formel I können in diesem Umwandlungen gegebenenfalls in geschützter Form vorliegen.

Weiter kann in einem Produkt der Formel I, welches im Rest Ar eine Hydroxygruppe aufweist, diese Gruppe in Niederalkoxy, Niederalkenyloxy oder niederes Cycloalkyl-niederalkoxy umgewandelt werden. Das Produkt oder sein Alkalimetall-, z.B. Natriumsalz, wird dabei mit einem reaktionsfähigen Ester eines Alkohols, der die genannten Reste einführt, umgesetzt. Wenn nötig können in diesen Umsetzungen die weiteren reaktionsfähigen Substituenten des Produktes der Formel I in geschützter Form vorliegen.

Ferner können gegebenenfalls geschützte Verbindungen der Formel I oder Zwischenprodukte, worin X Sauerstoff bedeutet, durch schwefeleinführende Reagentien wie z.B. Phosphorpentasulfid in Verbindungen umgewandelt werden, worin X Schwefel bedeutet.

Bezüglich der oben aufgeführten Reaktionen bedeutet der Ausdruck "geschützt" potentiell reaktionsfähige Substituenten, z.B. Amino-, Hydroxy- und andere störende Gruppen, in Uebereinstimmung mit in der Technik wohlbekannten Schutztechniken, z.B. wie unten illustriert, in geeigneter Weise so zu schützen, dass Nebenreaktionen vermieden werden. Dies kann dadurch erreicht werden, dass solche Substituenten vor einer gewünschten Reaktion geschützt und danach oder während der Reaktion eines Verfahrens, falls notwendig, die Schutzgruppen abgespalten

werden, wodurch die gewünschten Verbindungen, z.B. solche der Formel I, erhalten werden.

Daher kann in allen Fällen, wo OR Hydroxy bedeutet oder Ar eine freie Hydroxygruppe trägt, eine solche Hydroxygruppe als Ester, z.B. als Acylderivat, etwa als Niederalkanoyl-, Benzyloxycarbonyl- oder Niederalkoxycarbonylester; oder als Aether, zum Beispiel als 2-Tetrahydropyranyl- oder Benzyläther, geschützt werden.

In ähnlicher Weise kann eine freie basische Aminogruppe oder die ($-NR_4-$)-Gruppe, wenn sie wenigstens 1 Wasserstoff am Stickstoff trägt, als leicht zu spaltendes Amid, zum Beispiel als ein Acylderivat, etwa als Benzyloxycarbonyl-(Carbobenzyloxy)- oder als t-Butyloxycarbonylderivat, oder mit irgendeiner anderen leicht abspaltbaren N-Schutzgruppe, wie sie gewöhnlich in der Peptidchemie Verwendung findet, geschützt werden.

In einer entsprechend geschützten Verbindung der Formel I oder einem Zwischenprodukt, in welchen eine oder mehrere funktionelle Gruppen geschützt sind, können die geschützten funktionellen Gruppen, z.B. Amino- oder Hydroxygruppen, in an sich bekannter Weise freigesetzt werden, zum Beispiel durch Solvolyse, insbesondere saure Hydrolyse, oder durch Reduktion, insbesondere Hydrogenolyse.

Die oben genannten Reaktionen werden nach an sich bekannten Methoden, in Gegenwart oder Abwesenheit von Verdünnungsmitteln, vorzugsweise solchen, welche gegenüber den Reagenzien inert sind und diese lösen, Katalysatoren, Kondensations- oder anderen oben genannten Mitteln, und/oder in einer inerten Atmosphäre, unter Kühlung, bei Zimmertemperatur oder bei erhöhter Temperatur, vorzugsweise beim Siedepunkt des verwendeten Lösungsmittels, bei normalem oder erhöhtem Druck, durchgeführt.

Die Erfindung betrifft ebenfalls Abänderungen des vorliegenden Ver-

BAD ORIGINAL

fahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder optisch reinen Antipoden verwendet wird. Immer wenn gewünscht, werden die oben genannten Verfahren erst durchgeführt, nachdem alle potentiell störenden, reaktionsfähigen funktionellen Gruppen in geeigneter Weise geschützt sind, wie oben und in den Beispielen illustriert.

In den genannten Reaktionen werden vorteilhafterweise solche Ausgangsstoffe verwendet, welche zu den im vorstehenden als besonders bevorzugt beschriebenen Verbindungen führen. Die bevorzugten Ausgangsstoffe der Formeln VII und VIII sind die jeweiligen cis-Isomeren.

Die Erfindung betrifft auch neue Ausgangsstoffe und Verfahren zu ihrer Herstellung.

Abhängig von den gewählten Ausgangsstoffen und Methoden können die neuen Verbindungen als eines der möglichen Isomeren oder als Isomerengemisch vorliegen, zum Beispiel als reine geometrische Isomeren (cis oder trans), als reine optische Isomeren wie etwa Antipoden oder als Mischungen aus optischen Isomeren wie etwa Racematen oder als Mischungen aus geometrischen Isomeren.

Erhaltene geometrische oder diastereomere Isomerengemische der obigen Verbindungen oder Zwischenprodukte können nach an sich bekannten Methoden in die einzelnen racemischen oder optisch aktiven Isomeren getrennt werden, z.B. durch fraktionierte Destillation, Kristallisation und/oder Chromatographie. Racemische Produkte können gleichfalls in die optischen Antipoden gespalten werden, z.B. durch Trennung ihrer diastereomeren Salze, wie gemäss J.Org.Chem. 43, 3803 (1978), z.B. durch fraktionierte Kristallisation von d- und l-(Tartraten, Mande-

BAD ORIGINAL

laten, Camphersulfaten oder 1-Naphthyl-1-äthylisocyanaten) von Verbindungen, die eine basische, salzbildende Gruppe haben, oder von d- und ℓ-(α-Methylbenzylamin, Cinchonidin, Chinin, Chinidin, Ephedrin, Dehydroabietylamin, Brucin oder Strychnin)-Salzen von Verbindungen, die eine saure, salzbildende Gruppe besitzen.

Vorteilhafterweise wird jeweils das wirksamere Isomere und/oder der wirksamere Antipode der erfindungsgemässen Verbindungen isoliert.

Die Verbindungen der vorliegenden Erfindung werden schliesslich entweder in freier oder in Form ihrer Salze erhalten. Jede erhaltene Base kann in ein entsprechendes Säureadditionssalz umgewandelt werden, vorzugsweise unter Verwendung einer therapeutisch annehmbaren Säure oder eines Anionenaustauschers. Erhaltene Salze können in die entsprechenden freien Basen umgewandelt werden, zum Beispiel unter Verwendung einer stärkeren Base, beispielsweise eines Metall- oder Ammoniumhydroxids oder eines basischen Salzes, z.B. eines Alkalimetallhydroxids oder -carbonats, oder eines Kationenaustauschers. Diese oder andere Salze, etwa Pikrate, können auch zur Reinigung der erhaltenen Basen verwendet wreden, indem man die Basen in Salze überführt, diese abtrennt und reinigt, und aus den Salzen wiederum die Base freisetzt. Infolge der engen Beziehung zwischen den freien Verbindungen und ihren Salzen sind in diesem Zusammenhang unter freien Verbindungen sinn- und zweckmässig gegebenenfalls immer auch die entsprechenden Salze zu verstehen.

Die Verbindungen und ihre Salze können auch in Form ihrer Hydrate erhalten werden, oder sie können andere zur Kristallisation verwendete Lösungsmittel einschliessen.

Die vorliegende Erfindung betrifft im weiteren Verbindungen der Formel I und ihre therapeutisch annehmbaren, nichttoxischen Säureadditionssalze zur Verwendung als Medikamente, insbesondere als α- und β-adrenergische Blocker, zum Beispiel als hypotensive, antihypertensive und

herzbeeinflussende Wirkstoffe, z.B. zur Behandlung von erhöhtem Blutdruck, Herzstörungen und anderen Krankheiten, die auf eine andrenergische Rezeptorenblockade ansprechen; und insbesondere zur Verwendung bei der Herstellung pharmazeutischer Präparate, im besonderen Präparate, die eine andrenergische Rezeptorblockierungswirkung, ganz besonders eine antihypertensive oder kardiale Wirkung, haben.

Bei den erfindungsgemässen Präparaten handelt es sich um solche zur enteralen, wie oralen oder rektalen, sowie zur parenteralen Verabreichung an Säuger einschliesslich der Menschen. Die erfindungsgemässen pharmazeutischen Präparate enthalten mindestens eine Verbindung der Formel I, oder eines ihrer therapeutisch annehmbaren Salze als Wirkstoff, gegebenenfalls zusammen mit einem oder mehreren therapeutisch annehmbaren Trägerstoffen.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z.B. Lactose, Dextrose, Rohrzucker, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel. Injizierbare Präparate sind vorzugsweise isotonische Lösungen oder Suspensionen, und Suppositorien in erster Linie Fettemulsionen oder -suspensionen. Die pharmakologischen Präparate können sterilisiert sein und/oder Hilfsstoffe, z.B. Konservier-,

Stabilisier-, Netz- und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von etwa 0,1% bis etwa 75%, insbesondere von etwa 1% bis etwa 50%, des Aktivstoffes.

Insbesondere betrifft die vorliegende Erfindung Methoden zur Behandlung kardiovaskulärer Krankheiten. Die Dosierung des Wirkstoffs hängt von der Warmblüterspezies, deren Körpergewicht, Alter und dem individuellen Zustand sowie der Applikationsweise ab. Einzeldosen für Säuger mit einem Gewicht von ungefähr 50-70 kg können zwischen ungefähr 10-200 mg des aktiven Bestandteils enthalten.

Die folgenden Beispiele dienen zur Illustration der Erfindung; sie sollen jedoch diese in ihrem Umfang in keiner Weise einschränken. Temperaturen werden in Celsiusgraden angegeben. Wenn nicht anders angegeben, werden alle Verdampfungs-Vorgänge bei vermindertem Druck, vorzugsweise zwischen ungefähr 15 und 100 mm Hg, durchgeführt.

Beispiel 1: Ein Gemisch von 657,6 g 92%igem o-(Allyloxy)-phenoxymethyloxiran (US-Patent 3,483,221) und 512 g cis-1-(4-Aminocyclohexyl)-2-imidazolidinon wird in 6000 ml Isopropanol gerührt und unter Rückfluss 6 Stunden gekocht. Dieses heisse Reaktionsgemisch wird mit 162,1 g Fumarsäure versetzt und über Nacht bei Raumtemperatur gerührt. Das rohe Produkt wird abfiltriert und mit 1000 ml Isopropylalkohol gewaschen. Das erhaltene feuchte, feste Material wird zuerst aus 5000 ml Isopropylalkohol umkristallisiert und dann im Vakuum bei 65° bis zu einem konstanten Gewicht getrocknet. Man erhält ein weisses kristallines Produkt, welches bei 154-155° schmilzt. Nach Umkristallisation von 1781 g des erhaltenes Salzes aus 9700 ml wasserfreiem Aethanol erhält man das cis-1-(4-[3-(o-Allyloxyphenoxy)-2-hydroxypropylamino]-cyclohexyl)-2-imidazolidinon-hemifumarat. F. 156-157°. Dies ist die cis-Verbindung der Formel III, worin $R_3$ Wasserstoff bedeutet und $R_7$ für Allyloxy steht, in ihrer Hemifumarat-Salzform.

Eine Lösung von 1575 g des obigen Salzes in 7875 ml Wasser wird mit einer Lösung von 4,537 mol Kaliumcarbonat in 790 ml Wasser basisch gemacht. Man erhält das cis-1-(4-[3-(o-Allyloxyphenoxy)-2-hydroxypropylamino]-cyclohexyl)-2-imidazolidinon als ein Oel. Dieses wird wie oben beschrieben in das Hemifumarat von höherem Reinheitsgrad umgewandelt.

Der Ausgangsstoff wird wie folgt hergestellt: Eine Lösung von 1800 g p-Aminoacetanilid in 7000 ml Dimethylformamid wird bei Raumtemperatur gerührt und dann in einem Eis-Wasserbad auf 10-15° gekühlt. Zu der gerührten, gekühlten Lösung lässt man 1440 g 2-Chloräthyl-isocyanat innerhalb 50 Minuten zufliessen, wobei man das Reaktionsgemisch bei einer Temperatur zwischen 20 und 25° hält. Das Gemisch wird bei Raumtemperatur 90 Minuten gerührt und innerhalb 20 Minuten mit 8000 ml Eiswasser versetzt. Man rührt es weitere 2 Stunden, filtriert das feste Material ab und wäscht es mit 4000 ml Wasser. Das rohe Produkt wird in 7000 ml 95%igem Alkohol aufgeschlämmt. Das feste Material wird abfiltriert und an der Luft bei Raumtemperatur über Nacht getrock-

BAD ORIGINAL

net. Man erhält den 1-(p-Acetamidophenyl)-3-(2-chloräthyl)-harnstoff, der unter Zersetzung bei 207-209° schmilzt.

Ein Gemisch von 1600 g 1-(p-Acetamidophenyl)-3-(2-chloräthyl)-harnstoff in 7200 ml Dimethylformamid wird in einer Stickstoffatmosphäre bei Raumtemperatur gerührt. Zu der erhaltenen Lösung lässt man innerhalb 90 Minuten eine Lösung von 400 g Natriummethoxid in 2000 ml wasserfreiem Methylalkohol zufliessen, wobei die Temperatur steigt. Das Reaktionsgemisch wird weitere 20 Minuten gerührt, dann auf 70-75° erhitzt und bei dieser Temperatur 1 Stunde gehalten. Dann lässt man das Reaktionsgemisch sich langsam, innerhalb ungefähr 3 Stunden, unter Rühren auf 32° abkühlen. Dann gibt man rasch 5000 ml Eiswasser dazu. Das Rühren wird bei Raumtemperatur 4 Stunden fortgesetzt und das Gemisch über Nacht bei Raumtemperatur stehen gelassen. Das feste Material wird abfiltriert, mit 3000 ml Wasser gewaschen und an der Luft ungefähr 2 Tage bei Raumtemperatur getrocknet. Man erhält das 1-(p-Acetamidophenyl)-2-imidazolidinon. F. 270-273°.

Ein Gemisch von 4213 g 1-(p-Acetamidophenyl)-2-imidazolidinon in 39000 ml Wasser und 2930 g 5%igem Rhodium-auf-Kohle-Katalysator (50% feucht vom Wasser) wird bei 40° hydriert. Das Hydrierungsgemisch wird filtriert und das Filtrat im Vakuum zur Trockene eingedampft. Das erhaltene feste Material wird an der Luft getrocknet und dann pulverisiert. Das Rohprodukt wird aus 12000 ml Aceton umkristallisiert. Man erhält das 1-(4-Acetamidocyclohexyl)-2-imidazolidinon als ein Gemisch von cis- und trans-Isomeren. F. 211-230°.

Eine Lösung von 3271 g Natriumhydroxid in 33000 ml Wasser wird mit 3271 g 1-(4-Acetamidocyclohexyl)-2-imidazolidinon versetzt. Das Gemisch wird gerührt und 4 Stunden unter Rückfluss gekocht. Das Reaktionsgemisch wird auf Raumtemperatur gekühlt und unter Rühren mit 3271 g Natriumchlorid versetzt. Das Reaktionsgemisch wird zehnmal mit insgesamt 93000 ml Methylenchlorid extrahiert. Der Methylenchlorid-Extrakt, der das Produkt enthält, wird im Vakuum auf ungefähr 5000 ml konzentriert. Diese Lösung wird mit 300 g wasserfreiem Magnesiumsulfat versetzt. Das Gemisch wird bei Raumtemperatur ungefähr 3 Stunden gerührt, filtriert und das Filtrat im Vakuum zur Trockene eingedampft. Der erhaltene Rückstand wird im Hochvakuum bis zur Gewichtskonstanz getrocknet. Man erhält das 1-(4-Aminocyclohexyl)-2-imidazolidinon als ein Isomerengemisch, welches nach Stehenlassen bei Raumtemperatur kristallisiert.

Ein Gemisch von 9500 ml wasserfreiem Aethylalkohol und 2323 g 1-(4-Aminocyclohexyl)-2-imidazolidinon wird zwecks Herstellung einer Lösung erhitzt. Die Lösung wird auf Raumtemperatur gekühlt und mit 9200 ml gesättigter Lösung von wasserfreiem Chlorwasserstoffgas in Essigsäureäthylester auf den pH-Wert 1 eingestellt. Die Temperatur des Gemisches steigt auf 60° und das Produkt kristallisiert aus. Das Gemisch wird auf 10° gekühlt, das Rohprodukt abfiltriert, mit 3500 ml wasserfreiem Aethylalkohol gewaschen und im Vakuum 2 Tage bei 50-60° getrocknet. Das rohe Hydrochlorid wird zweimal aus wasserfreiem Methanol-Aethyläther (ungefähr 1:1) umkristallisiert. Man erhält das cis-1-(4-Aminocyclohexyl)-2-imidazolidinon-monohydrochlorid. F. 327-328°.

Eine Lösung von 630 g cis-1-(4-Aminocyclohexyl)-2-imidazolidinon-monohydrochlorid und 155 g Natriummethoxid in 6000 ml wasserfreiem Methanol wird in einer Stickstoffatmosphäre bei Raumtemperatur ungefähr 24 Stunden gerührt. Das Reaktionsgemisch wird in einem Eisbad gekühlt und das Natriumchlorid abfiltriert. Das Methanolfiltrat wird im Vakuum zur Trockne eingedampft. Der Rückstand wird in 1700 ml Dichlormethan unter Erwärmen gelöst. Das Gemisch wird im Eisbad gekühlt und mit einem Hyphlo-Filter filtriert. Der Filterkuchen wird mit 750 ml Dichlormethan gewaschen. Das Dichlormethan-Filtrat wird im Vakuum zur Trockene eingedampft und der Rückstand im Hochvakuum bis zur Gewichtskonstanz getrocknet. Man erhält das cis-1(-4-Aminocyclohexyl)-2-imidazolidinon. F. 121-123°.

Beispiel 2:

a) Eine Lösung von (S)-[o-(Cyclopropylmethoxy)-phenoxy]-methyloxiran (25,77 g) und cis-1-(4-Aminocyclohexyl)-2-imidazolidinon (23,6 g, Beispiel 1) in 300 ml Isopropanol wird in einer Stickstoffatmosphäre unter Rühren 5 1/2 Stunden unter Rückfluss gekocht. Man lässt die Lösung bei Raumtemperatur 16 Stunden stehen und dampft sie dann unter vermindertem Druck ein. Der Rückstand wird in 300 ml Essigsäureäthylester gelöst und mit einer Lösung von Chlorwasserstoffgas in Essigsäureäthylester auf den pH-Wert 1 angesäuert. Den amorphen festen Niederschlag filtriert man ab und wäscht ihn mit Essigsäureäthylester. Das feste Material wird in warmem Wasser gelöst, die Lösung auf Raumtemperatur gekühlt, mit Essigsäureäthylester (2 x 100 ml) gewaschen, mit überschüssiger 10%iger wässeriger Natriumcarbonatlösung basisch gemacht und mit Essigsäureäthylester (2 x 200 ml) extrahiert. Der organische Extrakt wird mit 10%iger wässeriger Natriumcarbonatlösung und Wasser gewaschen. Die organische Schicht wird über wasserfreiem Natriumsulfat getrocknet und unter vermindertem Druck eingedampft. Der Rückstand wird kurz im Hochvakuum getrocknet. Man erhält ein viskoses Oel.

Das Oel wird in das Hemifumarat umgewandelt, indem man 40,86 g Oel in 350 ml heissem Isopropanol löst und mit Fumarsäure (5,3 g) versetzt. Die Lösung wird langsam auf Raumtemperatur gekühlt, wobei sich das Produkt auskristallisiert. Das Gemisch wird in einem Eisbad weiter gekühlt, das feste Material abfiltriert und mit kaltem Isopropanol gewaschen. Das feste Material wird im Hochvakuum bei 50° getrocknet, wobei man das rohe Hemifumaratsalz erhält. Dieses wird durch Behandlung mit Aethylalkohol gereinigt. Das chirale Produkt ist löslicher als das Racemat. Wiederholte Kristallisation und Beseitigung des Racemats (das weniger löslich ist) durch Abfiltrieren, führt nach Eindampfen des Filtrates zur Isolierung des (S)-cis-1-(4-[3-(o-Cyclopropylmethoxyphenoxy)-2-hydroxypropylamino]-cyclohexyl)-2-imidazolidinon-hemifumarats (Salz des S-Enantiomeren). F. 147-149°.
$[\alpha]_D^{25}$ = -8,41° (c = 1,35 in Methanol). Dieses Produkt ist das Salz der

cis-(S)-Verbindung der Formel III, worin $R_7$ Cyclopropylmethoxy bedeutet und $R_3$ Wasserstoff ist.

Der optische Reinheitsgrad wird durch NMR in Gegenwart eines chiralen Shift-Reagenses ermittelt und ist grösser als 95%.

b) In analoger Weise ergibt die Kondensation des (R)-[o-(Cyclopropylmethoxy)-phenoxy]-methyloxirans (33,69 g) mit cis-1-[4-Aminocyclohexyl]-2-imidazolidinon das (R)-cis-1-(4-[3-(o-Cyclopropylmethoxy)-2-hydroxypropylamino]-cyclohexyl)-2-imidazolidinon-hemifumarat (Salz des R-Enantiomeren der cis-Verbindung der Formel III, worin $R_3$ Cyclopropylmethoxy bedeutet). F. 150-152° (Zersetzung).
$[\alpha]_D^{25}$ = +8,26° (c = 1,36 in Methanol).

Der Ausgangsstoff wird wie folgt hergestellt: Ein Gemisch von 42,5 g (R)-Epichlorhydrin [beschrieben in J. Organic Chemistry 43, 4876 (1978)], 36,8 g o-(Cyclopropylmethoxy)-phenol und 164 ml Aceton und 10 ml Hexan wird mit 30,9 g pulverisiertem Kaliumcarbonat versetzt und unter Rühren in einer Stickstoffatmosphäre 42 Stunden unter Rückfluss gekocht. Das Gemisch wird auf Raumtemperatur gekühlt, das feste Material abgetrennt und mit Aceton gewaschen. Das Filtrat wird vorsichtig im Hochvakuum bei Raumtemperatur eingedampft. Der Rückstand wird in Aether (200 ml) gelöst, mit eiskalter 1-normaler Natriumhydroxidlösung (2 x 50 ml) und Wasser (2 x 50 ml) gewaschen. Die Lösung wird über wasserfreiem Natriumsulfat getrocknet, abfiltriert und unter vermindertem Druck eingedampft. Der Rückstand wird im Vakuum destilliert und die Hauptfraktion, die bei 108-121°/0,2 mmHg siedet, aufgefangen. Sie besteht aus (S)-[o-(Cyclopropylmethoxy)-phenoxy]-methyloxiran.
$[\alpha]_D^{25}$ = +10,10 (c = 2,4 in Methanol).

In analoger Weise erhält man ausgehend von (S)-Epichlorhydrin das (R)-[o-(Cyclopropylmethoxy)-phenoxy]-methyloxiran. Siedepunkt 120-127°/0,2 mmHg, $[\alpha]_D^{25}$ = -9,76° (c = 1,58 in Methanol.

Das (S)-[o-(Cyclopropylmethoxy)-phenoxy]-methyloxiran wird auch wie folgt hergestellt:

Ein Gemisch von (S)-Benzyl-2,3-epoxypropyl-äther (8,95 g),[beschrieben in Heterocycles, Vol. 16, 381, (1981)], o-Cyclopropylmethoxyphenol (8,95 g) in 36 ml Aceton und 4 ml Hexan wird mit pulverisiertem Kaliumcarbonat (6,9 g) versetzt und in einer Stickstoffatmosphäre unter Rühren 74 Stunden unter Rückfluss gekocht. Das Gemisch wird auf Raumtemperatur gekühlt, das feste Material abfiltriert und mit Aceton gewaschen. Das Filtrat wird unter vermindertem Druck eingedampft, der Rückstand in Aether gelöst, die Lösung zweimal mit Wasser gewaschen, über wasserfreiem Natriumsulfat getrocknet, filtriert und das Filtrat zur Trockene eingedampft. Das rohe Oel wird im Vakuum bei 199-212°/ 0,2 mmHg destilliert. Man erhält das (S)-1-Benzyloxy-3-[o-cyclopropylmethoxy)-phenoxy]-2-hydroxypropan.

Eine Lösung von (S)-1-Benzyloxy-3-[o-(cyclopropylmethoxy)-phenoxy]-2-hydroxypropan (10,97 g) in 100 ml Aethanol wird mit 3,5 g 10%igem Palladium-auf-Kohle Katalysator bei Raumtemperatur und 3,06 Atmosphären Druck bis zum Aufhören der Wasserstoffaufnahme (ungefähr 10 Minuten) hydriert. Der Katalysator wird abfiltriert, mit Aethanol gewaschen und das Filtrat unter vermindertem Druck eingedampft. Der erhaltene kristalline Rückstand wird aus 35 ml Aethanol umkristallisiert. Man erhält das (S)-3-[o-(Cyclopropylmethoxy)-phenoxy]-1,2-propandiol. F. 95-97°, $[\alpha]_D^{25}$ = 5,32° (c = 0,99 in Methanol).

Eine Lösung von 3-[o-(Cyclopropylmethoxy)-phenoxy]-1,2-propandiol (5,82 g) in 15 ml trockenem Pyridin wird in einer Stickstoffatmosphäre auf 5° gekühlt und mit p-Toluolsulfonylchlorid (4,7 g) unter Rühren, innerhalb 10 Minuten versetzt. Das Gemisch wird bei 5° 1 1/2 Stunden gerührt und dann über Nacht in einem Kühlschrank bei ungefähr 5° stehen gelassen. Das Gemisch wird mit Aether (15 ml) versetzt, eine weitere Stunde bei 5° stehen gelassen, das erhaltene feste Material filtriert und mit Aether gewaschen. Das Filtrat wird bei niedriger Temperatur zur Trockene eingedampft. Der Rückstand wird in Aether aufgenommen, die Lösung mit eiskaltem Wasser gewaschen und dann auf einmal mit gesättigter Lösung von Natriumhydrogencarbonat versetzt. Die Lösung wird über

wasserfreiem Natriumsulfat getrocknet, filtriert und unter vermindertem Druck und im Hochvakuum eingedampft. Man erhält das (S)-3-[o-Cyclopropylmethoxy)-phenoxy]-1,2-propandiol-1-tosylat als ein Oel.
$[\alpha]_D^{25}$ = -8,49° (c = 1,26 in Methanol).

Eine Lösung von 3-[o-(Cyclopropylmethoxy)-phenoxy]-1,2-propandiol-1-tosylat (8,1 g) in 25 ml Methanol und 12 ml Aether wird unter Rühren in einer Stickstoffatmosphäre,unter Kühlung auf 5°, mit 0,47 g Natrium in kleinen Stücken innerhalb 30 Minuten versetzt. Das Gemisch wird bei 5° zwei Stunden weiter gerührt. Durch Einleiten von Kohlendioxid wird der pH-Wert des Gemisches auf 8 eingestellt. Das Gemisch wird filtriert und das Filtrat in einem warmem Wasserbad unter vermindertem Druck eingedampft. Dann versetzt man es mit Aether und Eiswasser. Die Schichten werden getrennt, die organische Schicht wird wieder mit Wasser gewaschen, über wasserfreiem Natriumsulfat getrocknet und unter vermindertem Druck eingedampft. Das erhaltene rohe Material wird im Vakuum destilliert. Man erhält das (S)-[o-(Cyclopropylmethoxy)-phenoxy]-methyloxiran, welches mit dem oben beschriebenen Produkt identisch ist. Nach dem Stehenlassen bei Raumtemperatur ergibt das Produkt wachsige, niedrig schmelzende Kristalle.

<u>Beispiel 3</u>: In analoger Weise erhält man gemäss den vorher beschriebenen Methoden die folgenden racemischen Verbindungen der Formel II, worin R, $R_3$, $R_4$ und $R_5$ Wasserstoff bedeuten, X für Sauerstoff steht und $R_1$ und $R_2$ zusammen $-CH_2CH_2-$ bedeuten. Wenn nicht anders angegeben, werden alle Produkte als Hemifumarate erhalten.

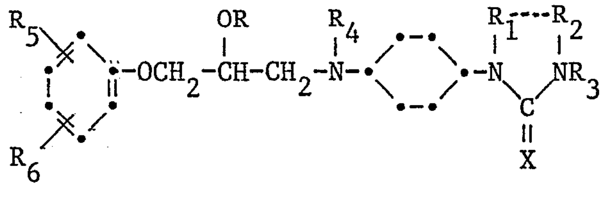

(II)

| Verbindung | $R_6$ | Das Isomere | Umkristallisiert aus | F. |
|---|---|---|---|---|
| 3/a | o-Methoxy | cis | iPrOH/$CH_3OH$ | 190-2° |
| 3/b | p-Propargyloxy | cis | 95% AetOH | 168° |
| 3/c | o-Propargyloxy | trans | - | 92-8° |
| 3/d | m-Propargyloxy | cis | AetOH | 177° |
| 3/e | p-Propargyloxy (1:1 Fumarat-Salz) | cis | iPrOH | 95-100° (iPrOH Solvat) |
| 3/f | o-Cyano | cis | AetOH | 192° Zers. |
| 3/g | o-Cyano | trans | AetOH | 215-6° |
| 3/h | o-(1-Pyrrolyl) | cis | AetOH | 144° Zers. |
| 3/i | o-(2-Pyrrolyl) | cis | AetOH | 130-4° |
| 3/j | o-Morpholino | cis | AetOH | 114-9° |
| 3/k | p-(2-Methoxyäthyl) | cis | iPrOH | 166-8° |
| 3/l | o-(Cyclopropylmethoxy) | trans | iPrOH | 178-80° |
| 3/m | o-(Cyclopropylmethoxy) | cis | AetOH | 168-170° |
| 3/n | o-Benzyloxy | cis | AetOH | 141-6° |
| 3/o | o-Carbamoyl | cis | - | 110° |
| 3/p | o-(2-Methylpropoxy) | cis | Aceton | 170-2° |
| 3/q | o-Cyclohexyl | cis | iPrOH/Aceton | 117-120 (Hydrat) |
| 3/r | o-Methoxycarbonyl | cis | iPrOH/Aceton | 135-8° |
| 3/s | O-Phenyl | cis | iPrOH | 146-8° |
| 3/t | p-Benzyloxy (HCl-Salz) | cis | AetOH | 159-61° |
| 3/u | o-Allyloxy | trans | AetOH | 159-50° |
| 3/v | o-Allyl | cis | iPrOH/Aceton | 158-9° |

iPrOH = Isopropylalkohol; AetOH = Aethylalkohol; Zers. = Zersetzung.

Die folgenden bekannten Ausgangsstoffe der Formel VI sind wie in der leicht verfügbaren chemischen Literatur beschrieben, hergestellt worden.

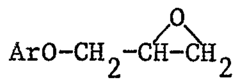
(VI)

| Ar | Ar |
|---|---|
| o-Methoxyphenyl | p-Benzyloxyphenyl |
| o-Propargyloxyphenyl | p-(2-Methoxyäthyl)-phenyl |
| m-Propargyloxyphenyl | o-(Benzyloxyphenyl |
| p-Propargyloxyphenyl | o-Carbamoylphenyl |
| o-Cyanophenyl | o-Biphenyl |
| o-(1-Pyrrolyl)-phenyl | o-Methoxycarbonylphenyl |
| o-(2-Pyrrolyl)-phenyl | o-Allylphenyl |
| o-Morpholinophenyl | |

Die folgenden Ausgangsstoffe werden wie folgt hergestellt:

1) Brenzcatechin (110 g) wird in 800 ml Isopropanol gelöst, zuerst mit einer Lösung von Natriumhydroxid (41 g) in 45 ml Wasser und dann mit Cyclopropylmethylchlorid (155 g, 70%rein) und Kaliumhydroxid (5 g) versetzt. Das Reaktionsgemisch wird in einer Stickstoffatmopshäre gerührt und 16 Stunden unter Rückfluss gekocht. Das Gemisch wird auf Raumtemperatur gekühlt, das feste Material abfiltriert und mit Isopropanol gewaschen. Das Filtrat wird unter vermindertem Druck eingedampft, der braune Rückstand in Toluol (600 ml) gelöst und mit 3-normaler Chlorwasserstoffsäure auf den pH-Wert 1 eingestellt. Die organische Phase wird mit Wasser (4 x 700 ml), mit wässeriger Natriumbisulfitlösung und Wasser (3 x 100 ml) gewaschen. Die organische Schicht wird über wasserfreiem Natriumsulfat getrocknet, durch basisches Aluminiumoxid (300 g) zwecks Beseitigung des noch vorhandenen Brenzcatechins filtriert und unter vermindertem Druck eingedampft. Der Rückstand wird im Vakuum durch eine Vigreux-Kolonne (versehen mit einem Rückfluss-Aufsatz) destilliert. Man erhält das 2-(Cyclopropylmethoxy)-phenol, welches bei 66-68°/0,1 mm siedet.

Eine Lösung von 6,92 g Natriumhydroxid in 28 ml Wasser wird mit 200 ml Aethanol, 24,6 g 2-(Cyclopropylmethoxy)-phenol und 83,25 g Epichlorhydrin versetzt. Das Reaktionsgemisch wird bei 25° unter Stickstoff 24 Stunden gerührt und dann 36 Stunden stehen gelassen. Das Gemisch wird vom Natriumchlorid abfiltriert und im Vakuum eingedampft. Der Rückstand wird zwischen Aether und Wasser verteilt. Die Aetherlösung wird noch dreimal mit Wasser gewaschen, getrocknet und eingedampft. Der Rückstand wird im Vakuum destilliert. Die zwischen 133 und 114°/ 0,5 mmHg siedende Fraktion entspricht dem o-(Cyclopropylmethoxy)-phenoxymethyloxiran der Verbindung der Formel VI, worin Ar o-(Cyclopropylmethoxy)-phenyl bedeutet.

2) Ersetzt man in den obigen Reaktionen das Cyclopropylmethylchlorid durch 1-Brom-2-methylpropan, so wird in analoger Weise das o-(2-Methylpropoxy)-phenoxymethyl-oxiran, die Verbindung der Formel VI, worin Ar o-(2-Methylpropoxy)-phenyl bedeutet, hergestellt.

3) Eine Lösung von 4,6 g Natriumhydroxid in 19 ml Wasser wird mit 150 ml Aethanol, 17,7 g 2-Cyclohexylphenol und 55,5 g Epichlorhydrin versetzt. Das Reaktionsgemisch wird über Nacht gerührt, filtriert und im Vakuum eingedampft. Der Rückstand wird im Vakuum destilliert. Die bei 127-133°/0,2 mmHg siedende Fraktion entspricht dem o-(Cyclohexyl)-phenoxymethyloxiran, der Verbindung der Formel VI, worin Ar o-(Cyclohexyl)-phenyl bedeutet.

4) Das trans-1-(4-Aminocyclohexyl)-2-imidazolidinon-hydrochlorid wird wie folgt hergestellt: Eine Lösung von rohem 1-(4-Aminocyclohexyl)-2-imidazolidinon-monohydrochlorid (66 g) (erhalten durch Eindampfen von Mutterlaugen aus der Herstellung und Reinigung des cis-1-(4-Aminocyclohexyl)-2-imidazolidinon-hydrochlorids im Beispiel 1 und welche hauptsächlich aus dem trans-Isomeren bestehen), in 1200 ml Aethanol und 600 ml Methanol wird bis zum Sieden erhitzt. Das unlösliche Material (5,5 g, hauptsächlich das cis-Isomere) wird abfiltriert und das heisse Filtrat in einem Eisbad gekühlt. Das beim Kühlen auskristallisierte Material wird abfiltriert und getrocknet. Man erhält 9,1 g

des festen Stoffes, der hauptsächlich aus dem trans-Isomeren besteht. F. 326-328° (Zersetzung). Das feste Material wird durch Auflösen in 100 ml heissem Methanol und Zugabe von 100 ml Aether umkristallisiert. Das nach Abkühlen auf Raumtemperatur erhaltene kristalline Material wird abgetrennt und getrocknet. Man erhält das trans-1-(4-Aminocyclohexyl)-2-imidazolidinon-monohydrochlorid. F. 328-329° (Zersetzung). Das Hydrochloridsalz wird in die freie Base, in das trans-1-(4-Aminocyclohexyl)-2-imidazolidinon, welches bei 168-170° schmilzt, umgewandelt.

Beispiel 4: Analog zu den in den Beispielen 1 und 2 beschriebenen Verfahren ergibt die Kondensation von cis-1-(4-Aminocyclohexyl)-3-methyl-2-imidazolidinon mit o-(Cyclopropylmethoxy)-phenoxymethyloxiran (s. Beispiel 3) das cis-1-(4-[3-(o-Cyclopropyl-methoxyphenyl)-2-hydroxypropylamino]-cyclohexyl)-3-methyl-2-imidazolidinon-hemifumaratsalz. F. 145-147°.

Der Ausgangsstoff wird wie folgt hergestellt: Man rührt in einer Stickstoffatmosphäre p-Nitroanilin (40 g) in 200 ml Dimethoxyäthan und versetzt es mit Chloräthylisocyanat (45,8 g) in drei Portionen innerhalb 4 1/2 Stunden. Nach der Zugabe der ersten Portion von ungefährt 65% wird die Lösung 1 1/2 Stunden unter Rückfluss gekocht. Dann gibt man weitere 20% dazu und kocht 1 1/2 Stunden unter Rückfluss. Nach der Zugabe des Restes vom Isocyanat setzt man das Kochen unter Rückfluss 1 1/2 Stunden weiter fort. Die dunkle Lösung wird auf Raumtemperatur abgekühlt, filtriert und das Filtrat unter vermindertem Druck eingedampft. Der Rückstand wird in Toluol aufgenommen und das Gemisch zwecks Beseitigung des nicht reagierten Chloräthylisocyanats wieder eingedampft. Der im wesentlichen feste Rückstand wird mit Essigsäureäthylester (400 ml) und Wasser (200 ml) behandelt und die Schichten werden getrennt. Die organische Schicht wird mit Wasser gewaschen, über wasserfreiem Natriumsulfat getrocknet, filtriert und unter vermindertem Druck eingedampft. Die Lösung des rohen 1-(p-Nitrophenyl)-3-(2-chloräthyl)-harnstoffs in 300 ml Methanol und 75 ml Dimethylformamid wird filtriert

und das Filtrat im nächsten Schritt direkt verwendet.

Die obige Lösung wird in einer Stickstoffatmosphäre gerührt und mit einer Lösung von Natrium-methoxid (hergestellt aus 7,4 g Natrium in 125 ml Methanol) innerhalb 20 Minuten versetzt. Das Reaktionsgemisch wird 2 Stunden unter Rückfluss gekocht, auf Raumtemperatur gekühlt, mit 300 ml Wasser verdünnt und gerührt. Der Niederschlag wird abfiltriert, zuerst mit Methanol/Wasser (1:1, 150 ml) und dann mit Wasser (100 ml) gewaschen. Das feste Material wird in Aethanol (150 ml) einige Minuten gerührt, filtriert und getrocknet. Man erhält das 1-(p-Nitrophenyl)-2-imidazolidinon. F. 243-245°.

Eine Lösung von 1-(p-Nitrophenyl)-2-imidazolidinon (25 g) in 450 ml Dimethylformamid wird unter Stickstoff gerührt und in kleinen Portionen mit Natriumhydrid (50%, 6,4 g) versetzt. Das Gemisch wird bei 50° 1 1/2 Stunden gerührt, auf Raumtemperatur gekühlt und mit Methyljodid (25 g) versetzt. Nach weiterem Rühren von 2 Stunden ist der pH-Wert des Reaktionsgemisches 9. Eine weitere Menge Methyljodid (4 g) wird innerhalb 6 Stunden zugegeben, bis der pH-Wert des Gemisches ungefähr 3 ist. Das Gemisch wird mit Wasser (150 ml) versetzt, der Niederschlag abfiltriert, zuerst mit Dimethylformamid/Wasser (1:1) und dann mit Wasser (2 x 150 ml) gewaschen und getrocknet. Man erhält das 1-(p-Nitrophenyl)-3-methyl-2-imidazolidinon. F. 207-208°.

Eine Lösung von 1-(p-Nitrophenyl)-3-methyl-2-imidazolidinon (12,45 g) in 150 ml Eisessig wird mit 6 g 5%igem Rhodium auf Aluminiumoxid versetzt und bei 50° und 3,06 Atmosphären Druck hydriert. Der Katalysator wird abfiltriert und das Filtrat unter vermindertem Druck eingedampft. Der Rückstand wird mit Wasser (25 ml) versetzt und unter Kühlung in einem Eisbad mit 50%iger wässeriger Natriumhydroxidlösung basisch gestellt. Nach Extraktion mit Methylenchlorid (5 x 70 ml), Trocknen der Extrakte mit wasserfreiem Natriumsulfat, Filtrieren und Eindampfen unter vermindertem Druck erhält man das 1-(4-Aminocyclohexyl)-3-methyl-2-imidazolidinon. Das Oel wird durch Auflösen in 100 ml Isopropanol und Hinzufügen einer gesättigten Lösung von Chlorwasser-

BAD ORIGINAL

in Essigsäureäthylester in das Hydrochloridsalz umgewandelt. Man gibt Aether (50 ml) dazu und das Salz kristallisiert nach Abkühlen auf Raumtemperatur aus. Das Gemisch wird in einem Kühlschrank weiter gekühlt. Der Niederschlag wird abfiltriert und getrocknet. Man erhält das cis-1-(4-Aminocyclohexyl)-3-methyl-2-imidazolidinon-hydrochlorid. F. 287-298°.

Das Hydrochloridsalz wird in die freie Base durch Lösen von 8,2 g Salz in 50 ml Wasser, Hinzufügen von 20 ml 50%iger wässeriger Natriumhydroxidlösung und Extraktion mit Methylenchlorid (5 x 40 ml) umgewandelt. Die Methylenchlorid-Lösung wird über wasserfreiem Natriumsulfat getrocknet, filtriert und eingedampft. Man erhält das cis-1-(4-Aminocyclohexyl)-3-methyl-2-imidazolidinon. F. 81-87°.

Beispiel 5: Ein Gemisch von cis-1-(4-[3-(o-Allyloxyphenoxy)-2-hydroxypropylamino]-cyclohexyl)-2-imidazolidinon (4,4 g) in 15 ml Isopropanol, 37,5%igem wässerigem Formaldehyd (0,671 g) und Ameisensäure (2,6 g) wird 19 Stunden unter Rückfluss gekocht. Die Lösung wird auf Raumtemperatur gekühlt, mit 1 ml konz. Chlorwasserstoffsäure versetzt und das Gemisch zur Trockene eingedampft. Der glasige Rückstand wird in 100 ml Wasser gelöst, mit überschüssiger 25%iger wässeriger Natriumhydroxidlösung basisch gemacht und mit Methylenchlorid (1 x 50 ml, 1 x 25 ml) extrahiert. Der Extrakt wird getrocknet, filtriert und eingedampft. Man erhält einen Rückstand, der durch präparative Dünnschichtchromatographie [auf Silicagel, unter Verwendung von Essigsäureäthylester-Methanol-Ammoniumhydroxid (90:5:5) als Eluent] gereinigt wird. Man erhält das cis-1-(4-[3-(o-Allyloxyphenoxy)-2-hydroxy-N-methylpropylamino]-cyclohexyl)-2-imidazolidinon als ein Oel. Das Hydrochloridsalz schmilzt bei 80° (Zersetzung). Es wird durch Auflösen von 2,3 g der Base in 50 ml Essigsäureäthylester, Hinzufügen einer gesättigten Lösung von Chlorwasserstoffgas in Essigsäureäthylester und Abtrennung des erhaltenen Salzes hergestellt.

Beispiel 6: Ein Gemisch von 3,0 g cis-1-(4-Aminocyclohexyl)-2-imidazolidinon, 3,2 g 3-Chlor-4-(oxiranylmethyloxy)-1,2,5-thiadiazol und 40 ml Isopropanol wird gerührt und 7 Stunden unter Rückfluss gekocht. Das Reaktionsgemisch wird filtriert und das Filtrat mit einer Lösung von wasserfreiem Chlorwasserstoff in Essigsäureäthylester angesäuert. Das Hydrochloridsalz fällt aus als amorphes festes Material. Nach Kühlen wird die obenstehende Lösung dekantiert und das rohe Hydrochloridsalz in 50 ml Wasser gelöst. Die Lösung wird filtriert, durch Hinzufügen von 2-normaler Natriumhydroxidlösung basisch gemacht und die freie Base mit Methylenchlorid extrahiert. Die Methylenchloridlösung wird getrocknet und im Vakuum eingedampft. Eine Lösung der freien rohen Base in 18 ml Isopropanol wird unter Rückfluss gekocht und mit 0,27 g Fumarsäure versetzt. Kristallisation des Produkts ergibt das cis-1-(N-[3-(4-Chlor-1,2,5-thiadiazol-3-yloxy)-2-hydroxypropylamino]-cyclohexyl)-2-imidazolidinon-hemifumarat. F. 163°. Das Lösungsmittel für die Umkristallisation ist Isopropanol.

Die Behandlung mit Morpholin bei 125-130° ergibt das cis-1-(N-[3-(4-Morpholinyl-1,2,5-thiadiazol-3-yloxy)-2-hydroxypropylamino]-cyclohexyl)-2-imidazolidinon.

Das als Ausgangsstoff verwendete 3-Chlor-4-(oxiranylmethyloxy)-1,2,5-thiadiazol wird wie in J.Med.Chem. 15, 651 (1972) beschrieben, hergestellt.

Beispiel 7: Die folgenden Verbindungen der Formel I, worin $R_1$ und $R_2$ zusammengenommen Aethylen bedeuten, $R_3$ und $R_4$ für Wasserstoff stehen, und X Sauerstoff bedeutet, können gemäss den in den vorhergehenden Beispielen beschriebenen Verfahren hergestellt werden.

BAD ORIGINAL

| Beispiel | Ar | R | Stereochemie |
|---|---|---|---|
| 7/a | 1-Naphthyl | H | cis |
| 7/b | 4-Indolyl | H | cis |
| 7/c | 3-Cyano-2-pyridyl | H | cis |
| 7/d | p-(Carbamoylmethyl)-phenyl | H | cis |
| 7/e | 3,4-Dihydro-2(1H)-quinolon-5-yl | H | cis |
| 7/f | 5,6,7,8-Tetrahydro-cis-6,7-dihydroxynaphth-1-yl | H | cis |
| 7/g | cis-o-(Cyclopropylmethoxy)-phenoxy | Benzoyl | cis |
| 7/h | cis-o-(Cyclopropylmethoxy)-phenoxy | Nicotinoyl | cis |
| 7/i | cis-o-(Allyloxy)-phenoxy | Acetyl | cis |

Die entsprechend substituierten, gegebenenfalls geschützten Oxiran-Ausgangsstoffe für die Verbindungen 7/a-7/f sind in der Literatur beschrieben.

Die Verbindungen 7/g-7/i können durch Acylierung von Verbindungen der Beispiele 1 und 3/m mit Benzoylchlorid, Nicotinoylchlorid bzw. Acetylchlorid, in einer Pyridinlösung hergestellt werden.

Beispiel 8: Eine Lösung von 3,7 g o-(Allyloxy)-phenoxymethyloxiran und 3,4 g 1-(4-Aminocyclohexyl)-3-äthylharnstoff in 50 ml Isopropylalkohol wird unter Rückfluss 3 1/2 Stunden gerührt. Die Lösung wird im Vakuum eingedampft. Das erhaltene Rohprodukt wird durch Säulenchromatographie auf Silicagel gereinigt, wobei man Methylenchlorid/Methylalkohol (zuerst 200:15, dann 1:1) als Eluent verwendet. Die langsam wandernde Hauptkomponente wird in 30 ml Isopropanol gelöst und mit 0,4 g Fumarsäure versetzt. Die Lösung wird zur Trockene eingedampft und mit 25 ml Aceton behandelt. Dieses wird dekantiert, der Rückstand in 25 ml Aceton und 2 ml Methylalkohol gelöst und die Lösung durch Filtrieren geklärt. Das auskristallisierte Produkt wird abfiltriert, mit wenig Aceton/Methylalkohol (9:1) gewaschen und bei 90° im Hochvakuum getrocknet.

Man erhält das 1-(4-[3-(o-Allyloxyphenoxy)-2-hydroxypropylamino]-cyclohexyl)-3-äthylharnstoff-hemifumarat. F. 159-160°.

Der Ausgangsstoff wird wie folgt hergestellt: Eine Lösung von 6,3 g 1-Aethyl-3-(p-nitrophenyl)-harnstoff in 75 ml Eisessig wird mit 3,2 g 5%igem Rhodium auf Aluminiumoxid versetzt und das Gemisch bei 3 Atmosphären Druck und 50° 3 1/2 Stunden hydriert. Der Katalysator wird abfiltriert und das Filtrat zur Trockene eingedampft. Der Rückstand wird mit 6-normaler Natriumhydroxidlösung basisch gemacht und mit Methylenchlorid (5 x 50 ml) extrahiert. Der Methylenchloridextrakt wird zur Trockene eingedampft, der Rückstand in 30 ml Isopropanol gelöst und mit einer Lösung von Chlorwasserstoff in Essigsäureäthylester auf den pH-Wert 1 eingestellt. Nach Hinzufügen von 60 ml Aether kristallisiert das 1-(4-Aminocyclohexyl)-3-äthylharnstoff-hydrochlorid aus. F. 230-232°.

Eine Lösung von 1,5 g 1-(4-Aminocyclohexyl)-2-äthylharnstoff-hydrochlorid in 20 ml Methylalkohol wird mit 0,36 g Natrium-methoxid behandelt. Nach üblicher Aufarbeitung erhält man den 1-(4-Aminocyclohexyl)-3-äthylharnstoff als ein Oel, welches in der obigen Kondensation direkt verwendet wird.

Beispiel 9: Eine Lösung von 2,67 g 1-(4-[3-(o-Cyclohexylphenoxy)-2-hydroxypropylamino]-phenyl)-2-imidazolidinon in 50 ml Essigsäure wird mit 1,75 g 5%igem Rhodium auf Aluminiumoxid versetzt und bei 50° und 3 Atmosphären Druck 5 Stunden hydriert. Nach Abkühlen auf 20° wird der Katalysator abfiltriert und das Filtrat zur Trockene eingedampft. Der Rückstand wird in 50 ml Wasser gelöst, die Lösung unter Kühlen in einem Eisbad mit 6-normaler wässeriger Natriumhydroxidlösung basisch gemacht und mit 75 ml Methylenchlorid extrahiert. Der Methylenchloridextrakt wird zweimal mit 25 ml Wasser gewaschen, über wasserfreiem Natriumsulfat getrocknet, filtriert und zur Trockene eingedampft. Man erhält ein dunkles Oel, welches in 30 ml Essigester gelöst und mit einer Lösung von Chlorwasserstoffgas in Essigsäureäthylester auf den

pH-Wert 1 eingestellt wird. Die obenstehende Lösung wird vom gummiartigen Niederschlag dekantiert und das Gummi in Wasser gelöst. Die wässerige Lösung wird mit Essigsäureäthylester gewaschen, die wässerige Schicht mit 10%iger wässeriger Natriumcarbonatlösung basisch gemacht und zweimal mit 25 ml Essigsäureäthylester extrahiert. Der Essigsäureäthylesterextrakt wird zweimal mit Wasser gewaschen, über wasserfreiem Natriumsulfat getrocknet, filtriert und im Vakuum eingedampft. Man erhält ein viskoses Oel. Man löst 0,94 ml des Oels in 10 ml heissem Aceton und versetzt es mit 0,13 g Fumarsäure. Es fällt ein Gummi aus, welches durch Zugabe von 2 ml Methylalkohol gelöst und mit 0,5 ml Wasser versetzt wird. Das auskristallisierte Produkt wird abfiltriert und im Vakuum bei 45° getrocknet. Man erhält das cis-1-(4-[3-(o-Cyclohexylphenoxy)-2-hydroxypropylamino]-cyclohexyl)-2-imidazolidinon-hemifumarat-hydrat, welches bei 117-120° schmilzt. Das Produkt ist mit demjenigen des Beispiels 3/q identisch.

Der Ausgangsstoff wird wie folgt hergestellt: Ein Gemisch von 10 g 1-(p-Acetamidophenyl)-2-imidazolidionon (s. Beispiel 1), 10 g Natriumhydroxid und 100 ml Wasser wird 4 Stunden unter Rückfluss gekocht. Nach Abkühlen kristallisiert das Produkt aus, welches abgetrennt und aus Aethanol umkristallisiert wird. Man erhält das 1-(4-Aminophenyl)-2-imidazolidinon. F. 170-172°.

Eine Lösung von 3,24 g o-(Cyclohexyl)-phenoxymethyloxiran (s. Beispiel 3) und 2,65 g 1-(4-Aminophenyl)-2-imidazolidinon in 60 ml Isopropanol wird 18 Stunden unter Rückfluss gekocht. Das Reaktionsgemisch wird filtriert und das Filtrat im Vakuum eingedampft. Der Rückstand wird in 50 ml Essigsäureäthylester gelöst und mit einer Lösung von Chlorwasserstoffgas in Essigsäureäthylester auf den pH-Wert 1 eingestellt. Die obenstehende Lösung wird vom erhaltenen dunklen Gummi dekantiert und der Rückstand mit Essigsäureäthylester und 5%iger wässeriger Natriumcarbonatlösung behandelt. Die Aethylacetatlösung wird abgetrennt, mit 10%iger wässeriger Natriumcarbonatlösung und zweimal mit Wasser gewaschen. Die Lösung wird über wasserfreiem Natriumsulfat getrocknet, mit

Aktivkohle behandelt, filtriert und im Vakuum eingedampft. Man erhält ein dunkles viskoses Gummi, welches auf einer Silicagelsäule chromatographiert und mit Methylenchlorid/Methylalkohol eluiert wird. Man erhält das 1-(4-[3-(o-Cyclohexylphenoxy)-2-hydroxypropylamino]-phenyl)-2-imidazolidinon, welches im nächsten Schritt direkt verwendet wird.

Beispiel 10: Herstellung von 10000 Tabletten mit einem Gehalt von je 10 mg der aktiven Substanz des Beispiels 1:

Bestandteile:

| | |
|---|---|
| Cis-1-(4-[3-(o-Allyloxyphenoxy)-2-hydroxypropylamino]-cyclohexyl)-2-imidazolidinon-hemifumarat | 100 g |
| Milchzucker | 1157 g |
| Maisstärke | 75 g |
| Polyäthylenglykol 6000 | 75 g |
| Talkpulver | 75 g |
| Magnesiumstearat | 18 g |
| gereinigtes Wasser | q.s. |

Verfahren:

Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyäthylenglykol in 150 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten mit 6,4 mm Durchmesser, welche eine Bruchrille aufweisen, gepresst.

Beispiel 11: Herstellung von 10000 Kapseln mit einem Gehalt von je 10 mg der aktiven Substanz des Beispiels 2.

Bestandteile:

| | |
|---|---|
| (S)-cis-1-(4-[3-(o-Cyclopropylmethoxyphenoxy)-2-hydroxypropylamino]-cyclohexyl)-2-imidazolidinon-hemifumarat | 100 g |
| Milchzucker | 1800 g |
| Talkpulver | 100 g |

Verfahren:

Sämtliche pulverigen Bestandteile werden mit einem Sieb von 0,6 mm Maschenweite gesiebt. Dann wird der Wirkstoff zuerst mit Talk und darauffolgend mit Milchzucker in einem geeigneten Mischer homogenisiert. Kapseln Nr. 3 werden mit je 200 mg des Gemisches mit einer Füllmaschine gefüllt.

In analoger Weise werden Tabletten oder Kapseln von anderen erfindungsgemässen Verbindungen, z.B. solchen, die in den weiteren Beispielen hier illustriert sind, hergestellt.

Beispiel 12: Eine Lösung von 3,7 g cis-1-(4-Aminocyclohexyl)-2-imidazolidinon und 4,7 g p-(Cyclopropylmethoxy)-phenoxymethyloxiran in 50 ml Isopropanol wird 6 Stunden unter Rückfluss gekocht. Man lässt die Lösung 16 Stunden bei Raumtemperatur stehen, gibt 1,17 g Fumarsäure dazu und erhitzt sie 30 Minuten unter Rückfluss. Es fällt ein Niederschlag aus. Das Reaktionsgemisch wird auf Raumtemperatur gekühlt, das Produkt abfiltriert, mit Isopropanol gewaschen und bei 65° im Vakuum getrocknet. Man erhält das cis-1-(4-[3-(p-Cyclopropylmethoxyphenoxy)-2-hydroxypropylamino]-cyclohexyl)-2-imidazolidinon-hemifumarat. F. 163-165°.

Der Ausgangsstoff wird wie folgt hergestellt: Ein Gemisch von 11,0 g Hydrochinon, 80 ml Isopropanol, 4,1 g Natriumhydroxid in 4,5 ml Wasser, 9,9 g Cyclopropylmethylchlorid und 0,5 g Kaliumjodid wird 12 Stunden unter Rückfluss gekocht. Das Lösungsmittel wird abgedampft und der Rückstand mit Aether extrahiert. Der Aetherextrakt wird nacheinander mit Wasser, Natriumbicarbonatlösung, verdünnter Natriumbisulfitlösung,

Wasser und Natriumchloridlösung gewaschen und getrocknet. Das nach Eindampfen des Lösungsmittels erhaltene Rohprodukt wird durch präparative Chromatographie [zuerst unter Verwendung von Toluol und dann von Toluol-Essigsäureäthylester (90:5 bis 85:15) als Eluent] gereinigt. Man erhält das (p-Cyclopropylmethoxy)-phenol. F. 49-52°.

Ein Gemisch von 0,46 g Natriumhydroxid in 1,8 ml Wasser und 14 ml Aethanol, 1,64 g p-(Cyclopropylmethoxy)-phenol und 5,54 g Epichlorhydrin wird in einer Stickstoffatmosphäre bei Raumtemperatur 24 Stunden gerührt und bei Raumtemperatur 2 1/2 Tage stehen gelassen. Das Lösungsmittel wird im Vakuum abgedampft, der Rückstand in Aether aufgenommen, mit Wasser und Natriumchloridlösung gewaschen. Die Lösung wird über wasserfreiem Natriumsulfat getrocknet, filtriert und unter vermindertem Druck eingedampft. Man erhält das o-(Cyclopropylmethoxy)-phenoxymethyloxiran, welches in der Kondensation direkt verwendet wird.

<u>Beispiel 13</u>: Eine Suspension von 3,8 g Lithiumaluminiumhydrid in 100 ml Tetrahydrofuran wird mit 3,6 g konz. Schwefelsäure bei -5 bis -10° tropfenweise versetzt. Das Gemisch wird 1 Stunde bei -5° weiter gerührt und dann unter Rühren innerhalb 5 bis 10 Minuten mit 6,5 g 3-(o-Allyloxyphenoxy)-2-hydroxy-N-[4-(imidazolidin-2-on-1-yl)-cyclohexyl]-propionamid versetzt. Man lässt das Reaktionsgemisch sich auf Raumtemperatur erwärmen, rührt es weitere 18 Stunden und schliesslich kocht es unter Rückfluss 1 Stunde. Nach Abkühlen wird das Gemisch zuerst mit Essigsäureäthylester, dann mit Wasser und Natriumhydroxid versetzt, filtriert und im Vakuum konzentriert. Der Rückstand wird in Essigsäureäthylester gelöst, die Lösung mit Wasser gewaschen, getrocknet und eingedampft. Der Rückstand wird in Isopropanol gelöst und unter Rühren und Kochen unter Rückfluss mit 0,9 g Fumarsäure versetzt. Nach Abkühlen kristallisiert das cis-1-(4-[3-(o-Allyloxyphenoxy)-2-hydroxypropylamino]-cyclohexyl)-2-imidazolidinon-hemifumaratsalz aus. Das Produkt ist demjenigen des Beispiels 1 identisch.

BAD ORIGINAL

Der Ausgangsstoff wird wie folgt hergestellt: Ein Gemisch von 62 g racemischer Chlormilchsäure, 82 g o-Allyloxyphenol und 90 g 50%iger Lösung von Natriumhydroxid in 500 ml Wasser wird unter Rühren 2 Stunden auf 80-90° erhitzt. Das heisse Gemisch wird mit konz. Chlorwasserstoffsäure angesäuert, wobei das Rohprodukt ausfällt. Nach Abkühlen filtriert man es ab und wäscht es mit Wasser. Die erhaltene 2-(o-Allyloxyphenoxy)-2-hydroxypropionsäure kann aus einem Gemisch von Alkohol und Wasser umkristallisiert werden.

Eine Lösung von 40 g Phosgen in 100 ml Tetrahydrofuran wird auf 10° gekühlt und zu einer Lösung von 48 g 3-(o-Allyloxyphenoxy)-2-hydroxypropionsäure in 250 ml Tetrahydrofuran bei Raumtemperatur unter Rühren gegeben. Man lässt das Reaktionsgemisch 18 bis 24 Stunden bei Raumtemperatur stehen und dampft es im Vakuum bei 40° ein. Der Rückstand wird in Aceton gelöst und auf -10° gekühlt. Man gibt dazu langsam unter Rühren eine Lösung von 37 g cis-1-(4-Aminocyclohexyl)-2-imidazolidinon und 34 g Natriumhydrogencarbonat in Wasser. Das Reaktionsgemisch wird 2 Stunden bei 20° gerührt, zwecks Beseitigung des Aceton-Hauptteils im Vakuum konzentriert und dann mit Methylenchlorid mehrmals extrahiert. Die Methylenchloridlösung wird mit Wasser gewaschen, getrocknet und im Vakuum eingedampft. Man erhält das 3-(o-Allyloxyphenoxy)-2-hydroxy-N-[4-(imidazolidin-2-on-1-yl)-cyclohexyl]-propionamid.

Beispiel 14: Eine Lösung von 2,5 g 3-(o-Allyloxyphenoxy)-2-hydroxypropylamin und 1,8 g 1-(4-Oxocyclohexyl)-2-imidazolidinon in 40 ml wasserfreiem Methanol wird mit Natriumcyanborhydrid (0,6 g), 0,6 ml Eisessig und 3 g Molekularsieb versetzt. Das Reaktionsgemisch wird 72 Stunden gerührt und der pH-Wert während dieser Zeit auf 4-5 gehalten. Das Molekularsieb wird abfiltriert, das Filtrat mit verdünnter Chlorwasserstoffsäure auf den pH-Wert 2 gestellt und das Methanol im Vakuum abgedampft. Der Rückstand wird mit Essigsäureäthylester extrahiert. Die wässerige Schicht wird mit 6-normaler wässeriger Natriumhydroxidlösung basisch gestellt und mehrmals mit Essigsäureäthylester extrahiert. Der Essigsäureäthylester wird im Vakuum abgedampft, der Rückstand in Isopropanol gelöst und durch Zugabe von 0,6 g Fumarsäure in das Hemifumaratsalz umgewandelt.

Das erhaltene Gemisch von cis- und trans-Isomeren von 1-(4-[3-(o-Allyloxyphenoxy)-2-hydroxypropylamino]-cyclohexyl)-2-imidazolidinon-hemifumarat wird durch Umkristallisation aus Isopropanol und/oder Aethanol aufgetrennt. Man erhält die cis-Verbindung des Beispiels 1.

Das Gemisch von cis- und trans-Isomeren des freien Amins kann zuerst durch präparative Chromatographie aufgetrennt und dann das freie cis-Amin in das Hemifumarat des Beispiels 1 umgewandelt werden.

Der 3-(o-Allyloxyphenoxy)-2-hydroxylpropylamin-Ausgangsstoff wird wie folgt hergestellt: Ein Gemisch von 50 g o-(Allyloxy)-phenoxymethyloxiran, 250 ml konz. Ammoniumhydroxid und 150 ml Isopropanol wird eine Woche bei Raumtemperatur gerührt. Das Reaktionsgemisch wird filtriert, das Filtrat auf ein kleines Volumen eingedampft, mit Essigsäureäthylester extrahiert, und der Extrakt eingedampft. Der Rückstand wird in heissem Isopropanol gelöst und mit 4-normaler Chlorwasserstoffsäure in Aethanol angesäuert. Man erhält das 3-(o-Allyloxyphenoxy)-2-hydroxypropylamin-hydrochlorid. F. 106-108°. Die freie Base wird aus diesem Hydrochloridsalz durch Behandlung mit Ammoniumhydroxid, Extraktion mit Methylenchlorid und Abdampfen des Lösungsmittels erhalten. F. 81-84°.

Der 1-(4-Oxocyclohexyl)-2-imidazolidinon-Ausgangsstoff wird wie folgt hergestellt: Ein Gemisch von 18,3 g 1-(4-Aminocyclohexyl)-2-imidoazolidinon als Gemisch von cis- und trans-Isomeren, 2,0 g Benzyltributylammoniumchlorid, 250 ml Essigsäureäthylester und 360 ml einer 10%igen wässerigen Kaliumhypochloritlösung [gemäss der von C.A. Meyers in J. Org.Chem. 26, 1046 (1961) beschriebenen Methode] wird 1 1/2 Stunden bei Raumtemperatur gerührt. Die organische Schicht wird abgetrennt und die wässerige Schicht mit Essigsäureäthylester extrahiert. Die vereinigten organischen Phasen werden mit Wasser, Natriumhydrogencarbonatlösung und verdünnter Chlorwasserstoffsäure gewaschen, getrocknet und zur Trockene eingedampft. Man erhält das 1-(4-Oxocyclohexyl)-2-imidazolidinon.

BAD ORIGINAL

Beispiel 15: Augentropfen enthaltend 0,1% der aktiven Verbindung:

| | |
|---|---|
| Cis-1-(4-[3-(o-Allyloxyphenoxy)-2-hydroxypropylamino]-cyclohexyl)-2-imidazolidinon-hemifumarat | 1,0 g |
| 0,01 M Citronensäure | 370,0 ml |
| 0,02 M Dinatriumphosphat | 620,0 ml |
| Dinatrium-metabisulfit | 1,0 g |
| Benzalkoniumchlorid | 0,1 g |
| Hydroxypropylmethylcellulose | 5,0 g |
| Destilliertes Wasser zum Auffüllen auf | 1000,0 ml. |

Die Bestandteile werden bei Raumtemperatur vermischt, die erhaltene Lösung filtriert, sterilisiert und unter sterilen Bedingungen in Tropfflaschen, die 15 ml Lösung enthalten, gefüllt.

Wirkung von Verbindungen der Erfindung auf den systolischen Blutdruck (BP, mm Hg) in spontan hypertensiven Ratten.

Gemäss der vorher beschriebenen Methode werden wache Ratten einzeln in Käfige gestellt. Zuerst werden die Kontrollwerte des Blutdrucks ermittelt und dann die einzelnen Testverbindungen einmal täglich, an zwei aufeinanderfolgenden Tagen, oral verabreicht.

Die Ergebnisse zeigen die Abnahme des Blutdrucks am zweiten Tag, 2 Stunden nach der Verabreichung des Wirkstoffs.

Im oben beschriebenen Test ergibt eine Auswahl der neuen Verbindungen die folgenden Werte.

Ergebnisse

| Verbindung des Beispiels | Dosis mg/kg p.o. | Abnahme des Bludrucks mm Hg |
|---|---|---|
| 1 | 50 | -79.3 |
| 3a | 50 | -39.3 |
| 3b | 30 | -47.8 |
| 3g | 50 | -33.0 |
| 3f | 50 | -11.0 |
| 3c | 50 | -30.0 |
| 3h | 50 | -68.0 |
| 3d | 50 | -52.3 |
| 3k | 50 | -17.6 |
| 2a | 30 | -75.5 |

Patentansprüche
(für alle benannten Länder ausser Oesterreich)

1. Verbindungen der allgemeinen Formel I

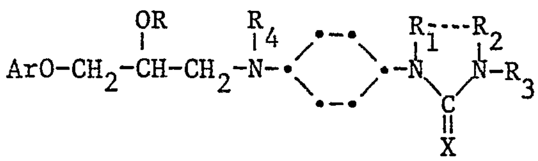

(I) ,

worin Ar einen (monocyclischen oder gegebenenfalls partiell hydrierten bicyclischen) carbocyclischen oder heterocyclischen, gegebenenfalls substituierten aromatischen Rest bedeutet, R für Wasserstoff, Alkanoyl oder Aroyl steht, $R_1$ und $R_2$ unabhängig voneinander Wasserstoff oder Niederalkyl oder $R_1$ und $R_2$ gemeinsam Alkylen mit 2 bis 7 Kohlenstoffatomen, welche die zwei Stickstoffatome durch 2 bis 4 Kohlenstoffatome trennen, bedeuten, $R_3$ und $R_4$ unabhängig voneinander für Wasserstoff oder Niederalkyl stehen, und X Oxo (O) oder Thio (S) bedeutet, und ihre Salze.

2. Verbindungen nach Anspruch 1, worin Ar gegebenenfalls substituiertes [Phenyl, Pyridyl, Naphthyl, Tetrahydronaphthyl, 3,4-Dihydro-1(2H)-naphthalenon, 3,4-Dihydro-2-(1H)chinolon, 3,4-Dihydro-1(2H)-isochinolon, Indolyl oder 1,2,5-Thiadiazolyl] bedeutet, und die anderen Symbole die im Anspruch 1 angegebenen Bedeutungen haben, und ihre Salze.

3. Verbindungen nach Anspruch 1, worin Ar gegebenenfalls durch einen bis drei Substituenten der Gruppe niederes (Alkyl, Alkenyl, Alkinyl, Alkanoyl, Mono- oder Dialkylamino, Alkanoylamino, Alkylsulfonylamino, Alkoxycarbonyl, Alkylcarbamoyl, Alkylsulfamoyl, Alkoxy, Alkylthio, Alkylsulfinyl, Alkylsulfonyl, Alkenyloxy oder Alkinyloxy), Hydroxy, Cyano, Halogen, Pyrrolyl, Amino, 5-, 6- oder 7-gliedriges (Alkylen-, Oxaalkylen-, Thiaalkylen)imino, Benzyloxy, Phenyl, 5-, 6- oder 7-gliedriges Cycloalkyl, Carbamoyl, Sulfamoyl oder durch 3- bis 7-gliedriges Cycloalkyl-, Phenyl-, Hydroxy-, Niederalkoxy-, Niederalkoxycarbonylamino-, Niederalkylthio-, Niederalkylsulfinyl-, Niederalkylsulfonyl- und durch Carbamoyl-substituiertes (Niederalkyl oder Niederalkoxy) substituiertes Phenyl bedeutet, R für Wasserstoff, Niederalkanoyl oder Aroyl steht; $R_1$ und $R_2$ Wasserstoff oder Niederalkyl, oder $R_1$ und $R_2$ gemeinsam Alkylen mit 2 bis 5 Kohlenstoffatomen bedeuten, $R_3$ und $R_4$ für Wasserstoff oder Niederalkyl stehen und X O oder S ist, und ihre Salze.

4. Verbindungen der Formel II

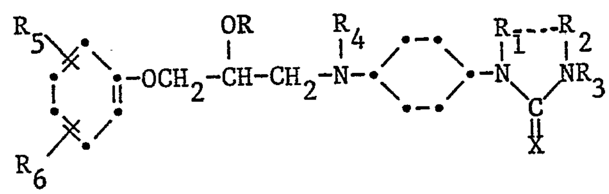

(II),

worin R Wasserstoff oder Niederalkanoyl bedeutet; jedes der Symbole $R_1$ und $R_2$ für Wasserstoff steht, oder $R_1$ und $R_2$ zusammen unverzweigtes Alkylen mit 2 bis 4 Kohlenstoffatomen bedeuten; $R_3$ und $R_4$ unabhängig voneinander Wasserstoff oder Niederalkyl bedeuten; $R_5$ und $R_6$ unabhängig voneinander Wasserstoff, niederes (Alkyl, Alkenyl oder Alkinyl), niederes (Alkoxy, Alkenyloxy oder Alkinyloxy), Hydroxy, Cyano, Halogen, Amino, niederes (Mono- oder Dialkylamino, Alkanoylamino oder Alkylsulfonylamino), niederes (Alkylthio, Alkylsulfinyl oder Alkylsulfonyl), Morpholino, 1- oder 2-Pyrrolyl, Phenyl, 5- bis 7-gliedriges Cycloalkyl, Niederalkanoyl, Niederalkoxycarbonyl, Carbamoyl oder Sulfamoyl bedeuten; $R_6$ auch Niederalkyl oder Niederalkoxy bedeutet, welche Reste durch einen Substituenten der Gruppe Cycloalkyl mit 3 bis 6 Kohlenstoffatomen, Phenyl, Niederalkoxy, Hydroxy, Niederalkoxycarbonylamino, Niederalkyl(thio, sulfinyl oder sulfonyl), oder durch Carbamoyl substituiert sein können; X O oder S bedeutet; und ihre Salze.

5. Verbindungen der Formel III

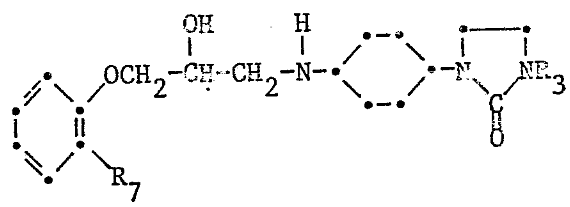

(III) ,

BAD ORIGINAL

worin $R_3$ Wasserstoff oder Niederalkyl bedeutet; $R_7$ für Cyan, Niederalkoxycarbonyl, Pyrrolyl, Morpholino, Alkenyloxy mit 3 bis 6 Kohlenstoffatomen, Alkinyloxy mit 3 bis 6 Kohlenstoffatomen oder Alkoxy mit 1 bis 3 Kohlenstoffatomen, welches durch Cyclopropyl substituiert ist, steht; und ihre Salze.

6. Verbindungen der Ansprüche 4 und 5, in welchen die zwei Substituenten des 1,4-Cyclohexylenrestes in cis-Stellung sind, und ihre Salze.

7. Verbindungen der im Anspruch 5 gezeigten Formel III, in welchen das die Hydroxygruppe tragende Kohlenstoffatom die S-Konfiguration aufweist, und ihre Salze.

8. Verbindungen der im Anspruch 5 gezeigten Formel III, worin $R_3$ Wasserstoff bedeutet, $R_7$ für Allyloxy, Propargyloxy oder Cyclopropylmethoxy steht, ihre Stereoisomeren und Enantiomeren, und Salze dieser Verbindungen.

9. Cis-1-(4-[3-(o-allyloxyphenoxy)-2-hydroxypropylamino]-cyclohexyl)-2-imidazolidinon und seine Salze.

10. Cis-1-(4-[3-(o-cyclopropylmethoxyphenoxy)-2-hydroxypropylamino)-cyclohexyl)-2-imidazolidinon, das S-Enantiomere,und ihre Salze.

11. Cis-1-(4-[3-(o-pyrrolylphenoxy)-2-hydroxypropylamino]-cyclohexyl)-2-imidazolidinon und seine Salze.

12. Pharmazeutische Präparate enthaltend Verbindungen der Ansprüche 1 bis 11, zusammen mit einem pharmazeutischen Trägermaterial.

13. Die in den Ansprüchen 1 bis 11 genannten Verbindungen zur Anwendung in einem Verfahren zur therapeutischen Behandlung des menschlichen oder tierischen Körpers.

14. Die in den Ansprüchen 1 bis 11 genannten Verbindungen mit kadiovaskulären Wirkungen.

15. Verwendung der in den Ansprüchen 1 bis 11 genannten Verbindungen zur Herstellung von pharmazeutischen Präparaten.

16. Verfahren zur Herstellung von den im Anspruch 1 genannten Verbindungen der Formel I und ihren Salzen, dadurch gekennzeichnet, dass man

a) eine Verbindung der Formel IV

$$ArO-CH_2-\overset{Y_1}{\overset{|}{CH}}-CH_2-Z_1 \qquad (IV)$$

mit einer Verbindung der Formel V

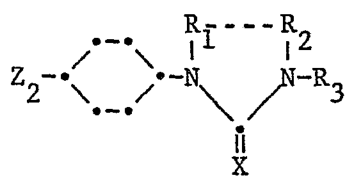

(V)

kondensiert, in welchen einer der Reste $Z_1$ und $Z_2$ eine raktionsfähige veresterte Hydroxygruppe und der andere die Gruppe $NHR_4$ bedeutet, und $Y_1$ für Hydroxy, Alkanoyloxy oder Aroyloxy steht; oder in welchen $Y_1$ und $Z_1$ zusammen eine Epoxygruppe darstellen, $Z_2$ die Aminogruppe $NHR_4$ bedeutet, und Ar, $R_1$, $R_2$, $R_3$, $R_4$ und X die oben angegebenen Bedeutungen haben; oder

b) eine Verbindung der Formel ArOH oder ihre Metallsalze, mit einer Verbindung der Formel X

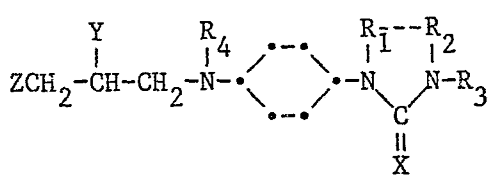

(X) ,

worin Z reaktionsfähiges verestertes Hydroxy und Y OR bedeutet; oder Z und Y zusammen eine Epoxygruppe bilden; und Ar, R, $R_1$, $R_2$, $R_3$, $R_4$ und X die oben definierten Bedeutungen haben; oder mit einer Verbin-

dung der Formel XI

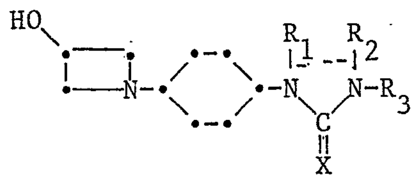 (XI) ,

worin $R_1$, $R_2$, $R_3$ und X die oben definierten Bedeutungen besitzen, kondensiert, oder

c) eine Verbindung der Formel XII

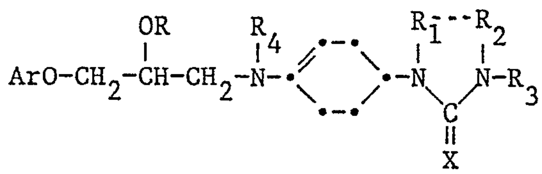 (XII)

oder, wenn $R_4$ Wasserstoff bedeutet, eines ihrer Tautomeren, reduziert, wobei Ar, R, $R_1$, $R_2$, $R_3$, $R_4$ und X die angegebenen Bedeutungen haben, oder

d) eine Verbindung der Formel XV

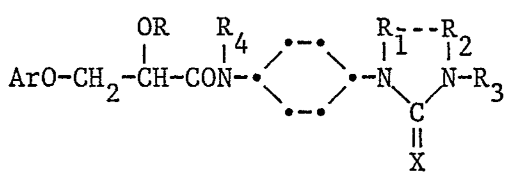 (XV)

reduziert, oder

e) eine Verbindung der Formel XVII

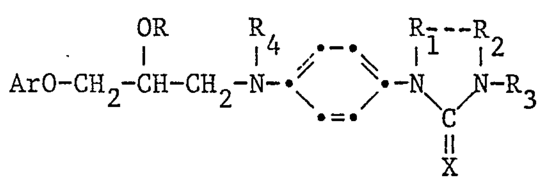 (XVII)

reduziert, oder

f) in einer Verbindung der Formel XVIII

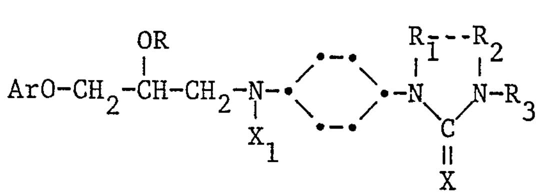 (XVIII) ,

worin $X_1$ einen abspaltbaren, durch Wasserstoff ersetzbaren Rest bedeutet, diesen durch Wasserstoff ersetzt, oder

g) eine Schiff'sche Base der Formel XIX

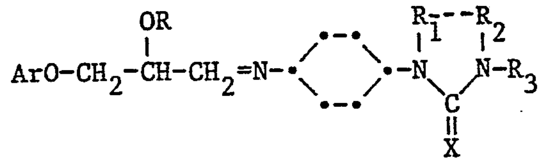

(XIX)

reduziert, oder

h) in einer Verbindung der Formel XX

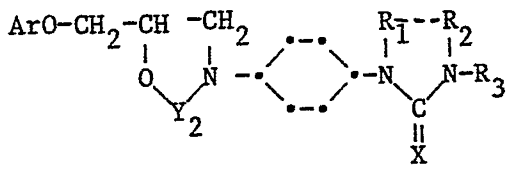

(XX)

oder in einem Salz davon, worin $Y_2$ einen zweiwertigen Rest, der durch zwei Wasserstoffatome ersetzbar ist, bedeutet, diesen Rest $Y_2$ abspaltet, oder

i) in einer Verbindung der Formel XXI

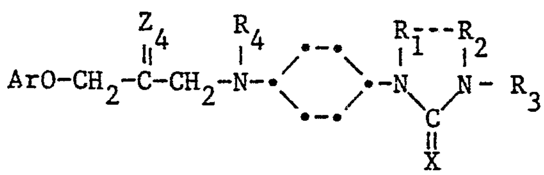

(XXI) ,

worin $Z_4$ ein Wasserstoffatom zusammen mit einer Gruppe $OZ_5$ bedeutet, worin $Z_5$ für einen abspaltbaren Rest steht; oder $Z_4$ ein Sauerstoffatom bedeutet; diesen Rest $R_4$ in ein Wasserstoffatom zusammen mit einer Hydroxygruppe umwandelt, und, wenn in allen diesen Verfahren a) bis i) notwendig, eine reaktionsfähige Gruppe, um Nebenreaktionen zu vermeiden, schützt, oder, wenn notwendig, eine in geschützter Form vorhandene Gruppe, während des Verfahrens oder nachfolgend, abspaltet, und/oder

1)wenn eine Verbindung der Formel I erwünscht ist, worin R Wasserstoff bedeutet, in einer Verbindung der Formel I, worin R Alkanoyl oder Aroyl bedeutet, diese Gruppen in Wasserstoff umwandelt, und/oder,

2) wenn eine Verbindung erwünscht ist, worin R Alkanoyl oder Aroyl bedeutet, in einer Verbindung der Formel I, worin R Wasserstoff ist, dieses in die genannten Gruppen überführt, und/oder,

3) wenn eine Verbindung erwünscht ist, worin $R_4$ Niederalkyl bedeutet, in eine Verbindung, worin $R_4$ Wasserstoff ist, einen genannten Rest einführt, und/oder,

4) wenn eine Verbindung der Formel I erwünscht ist, worin $R_3$ Niederalkyl bedeutet, in eine Verbindung, worin $R_3$ Wasserstoff ist, einen genannten Rest einführt, und/oder,

5) wenn eine Verbindung erwünscht ist, worin der Substituent der Gruppe Ar Alkyl ist, eine Verbindung, worin der Substituent Alkenyl oder Alkinyl ist, reduziert, und/oder,

6) wenn eine Verbindung erwünscht ist, worin der Substituent von Ar Carbamoyl ist, in einer Verbindung, worin der Substituent Cyano ist, diesen Rest in Carbamoyl umwandelt, und/oder,

7) wenn eine Verbindung erwünscht ist, worin die Gruppe Ar einen Hydroxy- oder Hydroxy-niederalkyl-Substituenten enthält, in einer Verbindung, in welcher diese Gruppen in geschützter Form sind, freisetzt, und/oder,

8) wenn eine Verbindung erwünscht ist, in welcher die Gruppe Ar einen Niederalkylsulfonylamino-Substituenten hat, in einer Verbindung, die einen Amino-Substituenten aufweist, diesen in den genannten Rest umwandelt, und/oder,

9) wenn eine Verbindung erwünscht ist, worin die Gruppe Ar einen Niederalkoxy-, Niederalkenyloxy- oder niederen Cycloalkyl-niederalkoxy-Substituenten hat, einen Hydroxy-Substituenten in die genannten Reste überführt, und/oder,

10) wenn eine Verbindung erwünscht ist, worin X ein Schwefelatom bedeutet, in einer Verbindung, worin X Sauerstoff ist, dieses in Schwefel umwandelt, und/oder, wenn erwünscht, eine erhaltene Verbindung der Formel I in eine andere Verbindung der Erfindung umwandelt,

und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

17. Die nach dem Verfahren des Anspruchs 16 erhältlichen Verbindungen.

18. Verbindungen der Formel

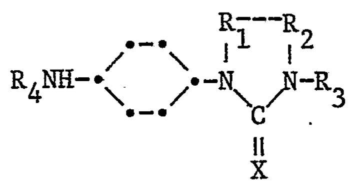 ,

worin $R_1$ und $R_2$ zusammen geradkettiges oder verzweigtes Alkylen mit 2 bis 7 Kohlenstoffatomen, welche die zwei Stickstoffatome durch 2 bis 4 Kohlenstoffatome trennen, bedeuten, $R_3$ und $R_4$ unabhängig voneinander für Wasserstoff oder Niederalkyl stehen, und X Oxo oder Thio bedeutet, und ihre Salze.

19. Verbindungen der Formel IX

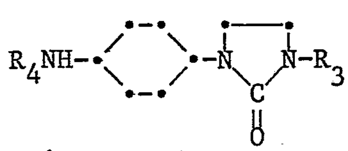 (IX)

oder ihre Stereoisomeren, worin $R_3$ und $R_4$ Wasserstoff oder Niederalkyl bedeuten, und ihre Salze.

20. Cis-1-(4-Aminocyclohexyl)-2-imidazolidinon und seine Salze.

FO 7.4/ZA/gs*

Patentansprüche
(für Oesterreich)

1. Verfahren zur Herstellung von neuen 3-(Ureidocyclohexylamino)-propan-1,2-diolderivaten der Formel I

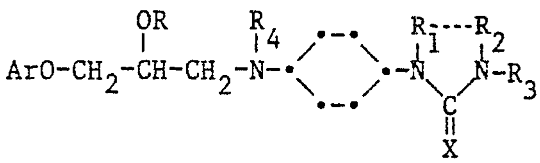

(I) ,

worin Ar einen (monocyclischen oder gegebenenfalls partiell hydrierten bicyclischen) carbocyclischen oder heterocyclischen, gegebenenfalls substituierten aromatischen Rest bedeutet, R für Wasserstoff, Alkanoyl oder Aroyl steht, $R_1$ und $R_2$ unabhängig voneinander Wasserstoff oder Niederalkyl oder $R_1$ und $R_2$ gemeinsam Alkylen mit 2 bis 7 Kohlenstoffatomen, welche die zwei Stickstoffatome durch 2 bis 4 Kohlenstoffatome trennen, bedeuten, $R_3$ und $R_4$ unabhängig voneinander für Wasserstoff oder Niederalkyl stehen, und X Oxo (O) oder Thio (S) bedeutet, und ihren Salzen, dadurch gekennzeichnet, dass man

a) eine Verbindung der Formel IV

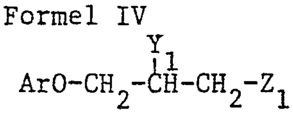

(IV)

mit einer Verbindung der Formel V

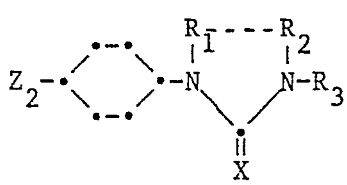

(V)

kondensiert, in welchen einer der Reste $Z_1$ und $Z_2$ eine raktionsfähige veresterte Hydroxygruppe und der andere die Gruppe $NHR_4$ bedeutet, und $Y_1$ für Hydroxy, Alkanoyloxy oder Aroyloxy steht; oder in welchen $Y_1$ und $Z_1$ zusammen eine Epoxygruppe darstellen, $Z_2$ die Aminogruppe $NHR_4$ bedeutet, und Ar, $R_1$, $R_2$, $R_3$, $R_4$ und X die oben angegebenen Bedeutungen haben; oder

b) eine Verbindung der Formel ArOH oder ihre Metallsalze, mit einer Verbindung der Formel X

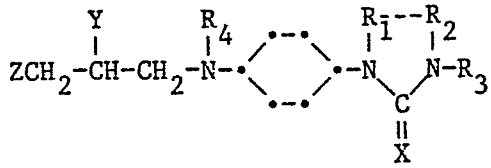 (X) ,

worin Z reaktionsfähiges verestertes Hydroxy und Y OR bedeutet; oder Z und Y zusammen eine Epoxygruppe bilden; und Ar, R, $R_1$, $R_2$, $R_3$, $R_4$ und X die oben definierten Bedeutungen haben; oder mit einer Verbindung der Formel XI

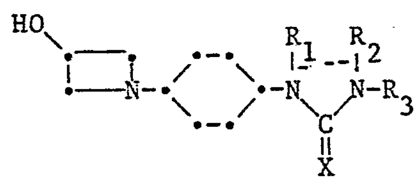 (XI) ,

worin $R_1$, $R_2$, $R_3$ und X die oben definierten Bedeutungen besitzen, kondensiert, oder

c) eine Verbindung der Formel XII

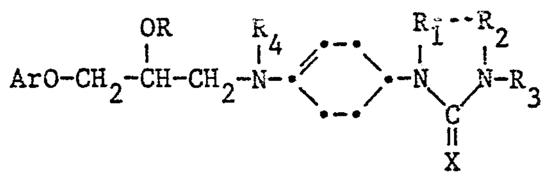 (XII)

oder, wenn $R_4$ Wasserstoff bedeutet, eines ihrer Tautomeren, reduziert, wobei Ar, R, $R_1$, $R_2$, $R_3$, $R_4$ und X die angegebenen Bedeutungen haben, oder

d) eine Verbindung der Formel XV

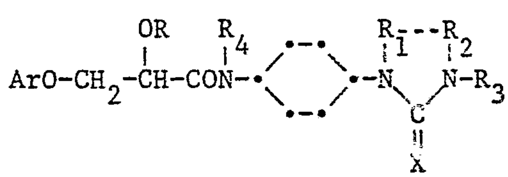 (XV)

reduziert, oder

e) eine Verbindung der Formel XVII

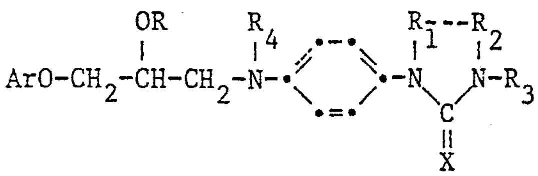

(XVII)

reduziert, oder

f) in einer Verbindung der Formel XVIII

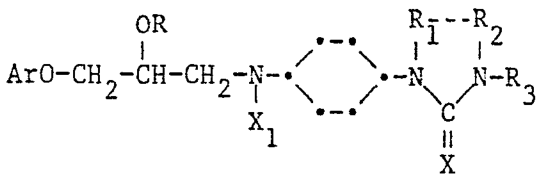

(XVIII) ,

worin $X_1$ einen abspaltbaren, durch Wasserstoff ersetzbaren Rest bedeutet, diesen durch Wasserstoff ersetzt, oder

g) eine Schiff'sche Base der Formel XIX

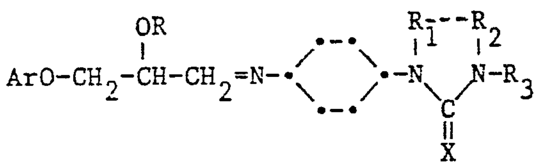

(XIX)

reduziert, oder

h) in einer Verbindung der Formel XX

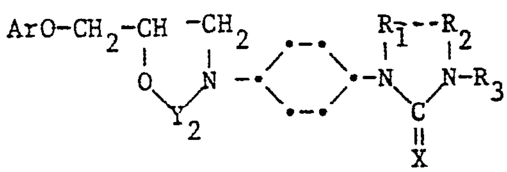

(XX)

oder in einem Salz davon, worin $Y_2$ einen zweiwertigen Rest, der durch zwei Wasserstoffatome ersetzbar ist, bedeutet, diesen Rest $Y_2$ abspaltet, oder

i) in einer Verbindung der Formel XXI

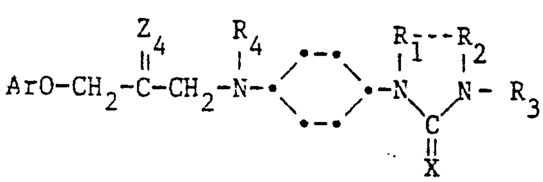

(XXI) ,

worin $Z_4$ ein Wasserstoffatom zusammen mit einer Gruppe $OZ_5$ bedeutet, worin $Z_5$ für einen abspaltbaren Rest steht; oder $Z_4$ ein Sauerstoffatom bedeutet; diesen Rest $R_4$ in ein Wasserstoffatom zusammen mit einer Hydroxygruppe umwandelt, und, wenn in allen diesen Verfahren a) bis i) notwendig, eine reaktionsfähige Gruppe, um Nebenreaktionen zu vermeiden, schützt, oder, wenn notwendig, eine in geschützter Form vorhandene Gruppe, während des Verfahrens oder nachfolgend, abspaltet, und/oder

1)wenn eine Verbindung der Formel I erwünscht ist, worin R Wasserstoff bedeutet, in einer Verbindung der Formel I, worin R Alkanoyl oder Aroyl bedeutet, diese Gruppen in Wasserstoff umwandelt, und/oder,

2) wenn eine Verbindung erwünscht ist, worin R Alkanoyl oder Aroyl bedeutet, in einer Verbindung der Formel I, worin R Wasserstoff ist, dieses in die genannten Gruppen überführt, und/oder,

3) wenn eine Verbindung erwünscht ist, worin $R_4$ Niederalkyl bedeutet, in eine Verbindung, worin $R_4$ Wasserstoff ist, einen genannten Rest einführt, und/oder,

4) wenn eine Verbindung der Formel I erwünscht ist, worin $R_3$ Niederalkyl bedeutet, in eine Verbindung, worin $R_3$ Wasserstoff ist, einen genannten Rest einführt, und/oder,

5) wenn eine Verbindung erwünscht ist, worin der Substituent der Gruppe Ar Alkyl ist, eine Verbindung, worin der Substituent Alkenyl oder Alkinyl ist, reduziert, und/oder,

6) wenn eine Verbindung erwünscht ist, worin der Substituent von Ar Carbamoyl ist, in einer Verbindung, worin der Substituent Cyano ist, diesen Rest in Carbamoyl umwandelt, und/oder,

7) wenn eine Verbindung erwünscht ist, worin die Gruppe Ar einen Hydroxy- oder Hydroxy-niederalkyl-Substituenten enthält, in einer Verbindung, in welcher diese Gruppen in geschützter Form sind, freisetzt, und/oder,

8) wenn eine Verbindung erwünscht ist, in welcher die Gruppe Ar einen Niederalkylsulfonylamino-Substituenten hat, in einer Verbindung, die einen Amino-Substituenten aufweist, diesen in den genannten Rest umwandelt, und/oder,

9) wenn eine Verbindung erwünscht ist, worin die Gruppe Ar einen Niederalkoxy-, Niederalkenyloxy- oder niederen Cycloalkyl-niederalkoxy-Substituenten hat, einen Hydroxy-Substituenten in die genannten Reste überführt, und/oder,

10) wenn eine Verbindung erwünscht ist, worin X ein Schwefelatom bedeutet, in einer Verbindung, worin X Sauerstoff ist, dieses in Schwefel umwandelt, und/oder, wenn erwünscht, eine erhaltene Verbindung der Formel I in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

2. Verfahren nach Anspruch 1, dadurch gekennzeichnet, dass man Verbindungen der Formel I, worin Ar gegebenenfalls substituiertes [Phenyl, Pyridyl, Naphthyl, Tetrahydronaphthyl, 3,4-Dihydro-1(2H)-naphthalenon, 3,4-Dihydro-2-(1H)chinolon, 3,4-Dihydro-1(2H)-isochinolon, Indolyl oder 1,2,5-Thiadiazolyl] bedeutet, und die anderen Symbole die im Anspruch 1 angegebene Bedeutung haben, und ihre Salze herstellt.

3. Verfahren nach Anspruch 3, dadurch gekennzeichnet, dass man Verbindungen der Formel I, worin Ar gegebenenfalls durch einen bis drei Substituenten der Gruppe niederes (Alkyl, Alkenyl, Alkinyl, Mono- oder Dialkylamino, Alkanoylamino, Alkylsulfonylamino, Alkoxycarbonyl, Alkylcarbamoyl, Alkylsulfamoyl, Alkoxy, Alkylthio, Alkylsulfinyl, Alkylsulfonyl, Alkenyloxy oder Alkinyloxy), Hydroxy, Cyano, Halogen,

Pyrrolyl, Amino, 5-, 6- oder 7-gliedriges (Alkylen-, Oxaakylen-, Thiaalkylen)imino, Benzyloxy, Phenyl, 5-, 6- oder 7-gliedriges Cycloalkyl, Carbamoyl, Sulfamoyl oder durch 3- bis 7-gliedriges Cycloalkyl-, Phenyl-, Hydroxy-, Niederalkoxy-, Niederalkoxycarbonylamino-, Niederalkylthio-, Niederalkylsulfinyl-, Niederalkylsulfonyl- und durch Carbamoyl-substituiertes (Niederalkyl oder Niederalkoxy) substituiertes Phenyl bedeutet, R für Wasserstoff, Niederalkanoyl oder Aroyl steht; $R_1$ und $R_2$ Wasserstoff oder Niederalkyl, oder $R_1$ und $R_2$ gemeinsam Alkylen mit 2 bis 5 Kohlenstoffatomen bedeuten, $R_3$ und $R_4$ für Wasserstoff oder Niederalkyl stehen und X O oder S ist und ihre Salze herstellt.

4. Verfahren nach Abspruch 1, dadurch gekennzeichnet, dass man Verbindungen der Formel II

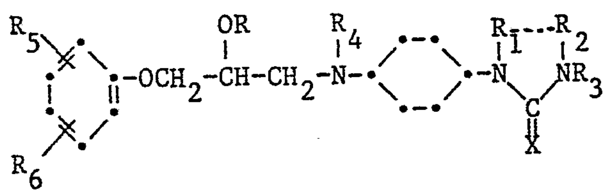

(II),

worin R Wasserstoff oder Niederalkanoyl bedeutet; jedes der Symbole $R_1$ und $R_2$ für Wasserstoff steht, oder $R_1$ und $R_2$ zusammen unverzweigtes Alkylen mit 2 bis 4 Kohlenstoffatomen bedeuten; $R_3$ und $R_4$ unabhängig voneinander Wasserstoff oder Niederalkyl bedeuten; $R_5$ und $R_6$ unabhängig voneinander Wasserstoff, niederes (Alkyl, Alkenyl oder Alkinyl), niederes (Alkoxy, Alkenyloxy oder Alkinyloxy), Hydroxy, Cyano, Halogen, Amino, niederes (Mono- oder Dialkylamino, Alkanoylamino oder Alkylsulfonylamino), niederes (Alkylthio, Alkylsulfinyl oder Alkylsulfonyl), Morpholino, 1- oder 2-Pyrrolyl, Phenyl, 5- bis 7-gliedriges Cycloalkyl, Niederalkanoyl, Niederalkoxycarbonyl, Carbamoyl oder Sulfamoyl bedeuten; $R_6$ auch Niederalkyl oder Niederalkoxy bedeutet, welche Reste durch einen Substituenten der Gruppe Cycloalkyl mit 3 bis 6 Kohlenstoffatomen, Phenyl, Niederalkoxy, Hydroxy, Niederalkoxycarbonylamino, Niederalkyl(thio, sulfinyl oder sulfonyl), oder durch Carbamoyl substituiert sein können; X O oder S bedeutet; und ihre Salze herstellt.

BAD ORIGINAL

5. Verfahren nach Anspruch 1, dadurch gekennzeichnet, dass man Verbindungen der Formel III

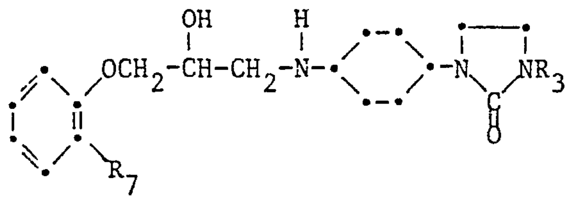

(III) ,

worin $R_3$ Wasserstoff oder Niederalkyl bedeutet; $R_7$ für Cyan, Niederalkoxycarbonyl, Pyrrolyl, Morpholino, Alkenyloxy mit 3 bis 6 Kohlenstoffatomen, Alkinyloxy mit 3 bis 6 Kohlenstoffatomen oder Alkoxy mit 1 bis 3 Kohlenstoffatomen, welches durch Cyclopropyl substituiert ist, steht; und ihre Salze herstellt.

6. Verfahren nach Anspruch 1, dadurch gekennzeichnet, dass man Verbindungen der Ansprüche 4 und 5, in welchen die zwei Substituenten des 1,4-Cyclohexylenrestes in cis-Stellung stehen, und ihre Salze herstellt.

7. Verfahren nach Anspruch 1, dadurch gekennzeichnet, dass man Verbindungen der im Anspruch 5 gezeigten Formel III, in welchen das die Hydroxygruppe tragende Kohlenstoffatom die cis-Konfiguration aufweist, und ihre Salze herstellt.

8. Verfahren nach Anspruch 1, dadurch gekennzeichnet, dass man Verbindungen der im Anspruch 5 gezeigten Formel III, worin $R_3$ Wasserstoff bedeutet, $R_7$ für Allyloxy, Propargyloxy oder Cyclopropylmethoxy steht, ihre Stereoisomeren und Enantiomeren, und Salze dieser Verbindungen herstellt.

9. Verfahren nach Anspruch 1, dadurch gekennzeichnet, dass man cis-1-(4-[3-(o-Allyloxyphenoxy)-2-hydroxypropylamino]-cyclohexyl)-2-imidazolidinon und seine Salze herstellt.

10. Verfahren nach Anspruch 1, dadurch gekennzeichnet, dass man cis-1-(4-[3-(o-Cyclopropylmethoxyphenoxy)-2-hydroxypropylamino]-cyclohexyl)-2-imidazolidinon, das S-Enantiomere und ihre Salze herstellt.

11. Verfahren nach Anspruch 11, dadurch gekennzeichnet, dass man cis-1-(4-[3-(o-Pyrrolylphenoxy)-2-hydroxypropylamino]-cyclohexyl)-2-imidazolidinon und seine Salze herstellt.

12. Verfahren nach Anspruch 1 zur Herstellung von Verbindungen der im Anspruch 1 gezeigten Formel I, und ihren Salzen, dadurch gekennzeichnet, dass man

a) eine Verbindung der Formel IV

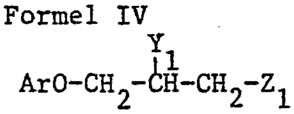 (IV)

mit einer Verbindung der Formel V

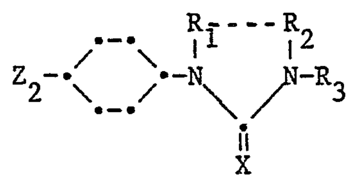 (V)

kondensiert, in welchen einer der Reste $Z_1$ und $Z_2$ eine raktionsfähige veresterte Hydroxygruppe und der andere die Gruppe $NHR_4$ bedeutet, und $Y_1$ für Hydroxy, Alkanoyloxy oder Aroyloxy steht; oder in welchen $Y_1$ und $Z_1$ zusammen eine Epoxygruppe darstellen, $Z_2$ die Aminogruppe $NHR_4$ bedeutet, und Ar, $R_1$, $R_2$, $R_3$, $R_4$ und X die oben angegebenen Bedeutungen haben; oder

b) eine Verbindung der Formel ArOH oder ihre Metallsalze, mit einer Verbindung der Formel X

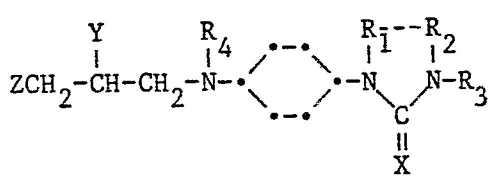 (X) ,

worin Z reaktionsfähiges verestertes Hydroxy und Y OR bedeutet; oder Z und Y zusammen eine Epoxygruppe bilden; und Ar, R, $R_1$, $R_2$, $R_3$, $R_4$ und X die oben definierten Bedeutungen haben; oder mit einer Verbin-

BAD ORIGINAL

dung der Formel XI

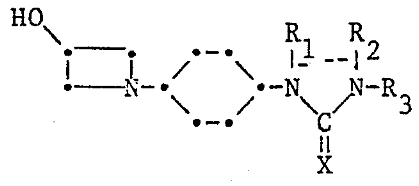

(XI) ,

worin $R_1$, $R_2$, $R_3$ und X die oben definierten Bedeutungen besitzen, kondensiert, oder

c) eine Verbindung der Formel XII

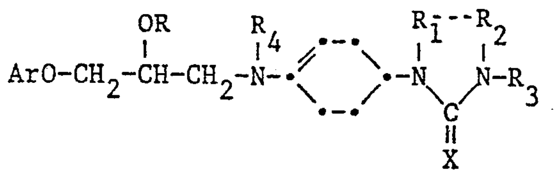

(XII)

oder, wenn $R_4$ Wasserstoff bedeutet, eines ihrer Tautomeren, reduziert, wobei Ar, R, $R_1$, $R_2$, $R_3$, $R_4$ und X die angegebenen Bedeutungen haben, oder

d) eine Verbindung der Formel XV

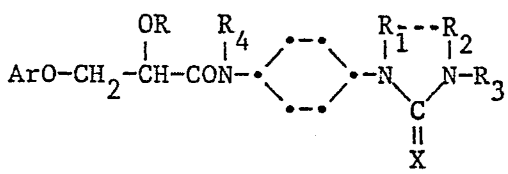

(XV)

reduziert, oder

e) eine Verbindung der Formel XVII

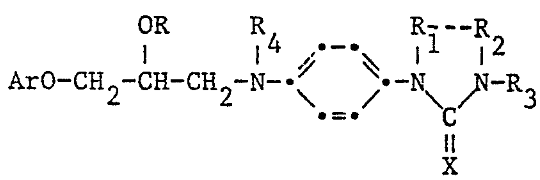

(XVII)

reduziert, und, wenn in allen diesen Verfahren a) bis e) notwendig, eine reaktionsfähige Gruppe, um Nebenreaktionen zu vermeiden, schützt, oder, wenn notwendig, eine in geschützter Form vorhandene Gruppe, während des Verfahrens oder nachfolgend, abspaltet, und/oder

1) wenn eine Verbindung der Formel I erwünscht ist, worin R Wasserstoff

bedeutet, in einer Verbindung der Formel I, worin R Alkanoyl oder Aroyl bedeutet, diese Gruppen in Wasserstoff umwandelt, und/oder,

2) wenn eine Verbindung erwünscht ist, worin R Alkanoyl oder Aroyl bedeutet, in einer Verbindung der Formel I, worin R Wasserstoff ist, dieses in die genannten Gruppen überführt, und/oder,

3) wenn eine Verbindung erwünscht ist, worin $R_4$ Niederalkyl bedeutet, in eine Verbindung, worin $R_4$ Wasserstoff ist, einen genannten Rest einführt, und/oder,

4) wenn eine Verbindung der Formel I erwünscht ist, worin $R_3$ Niederalkyl bedeutet, in eine Verbindung, worin $R_3$ Wasserstoff ist, einen genannten Rest einführt, und/oder,

5) wenn eine Verbindung erwünscht ist, worin der Substituent der Gruppe Ar Alkyl ist, eine Verbindung, worin der Substituent Alkenyl oder Alkinyl ist, reduziert, und/oder,

6) wenn eine Verbindung erwünscht ist, worin X ein Schwefelatom bedeutet, in einer Verbindung, worin X Sauerstoff ist, dieses in Schwefel umwandelt, und/oder, wenn erwünscht, eine erhaltene Verbindung der Formel I in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

13. Verfahren nach Anspruch 12, dadurch gekennzeichnet, dass man die in den Ansprüchen 2 bis 11 genannten Verbindungen und ihre Salze herstellt.

14. Verfahren zur Herstellung eines pharmazeutischen Präparates, gekennzeichnet durch die Verarbeitung eines erfindungsgemässen

Wirkstoffes nach einem der Ansprüche 1 bis 11, mit einem pharmazeutischen Trägermaterial.

15. Verfahren zur Herstellung eines pharmazeutischen Präparates, gekennzeichnet durch die Verarbeitung eines erfindungsgemässen Wirkstoffes nach einem der Ansprüche 12 und 13, mit einem pharmazeutischen Trägermaterial.